# EUROPÄISCHE PATENTSCHRIFT

(11) **EP 1 542 975 B1**
(45) Veröffentlichungstag und Bekanntmachung des Hinweises auf die Patenterteilung: **09.03.2016**
(21) Anmeldenummer: 03798150.3
(22) Anmeldetag: 08.09.2003
(51) Int. Cl.: C07D 221/18, C07D 311/78

(54) **9-CYANOSUBSTITUIERTE PERYLEN-3,4-DICARBONS UREMONOIMIDE**
9-CYANO-SUBSTITUTED PERYLENE-3,4-DICARBOXYLIC ACID MONOIMIDES
MONOIMIDES D'ACIDE PERYLENE-3,4-DICARBOXYLIQUE SUBSTITUES PAR DES GROUPES CYANO EN POSITION 9

(30) Priorität: 20.09.2002 DE 10243906
(43) Veröffentlichungstag der Anmeldung: 22.06.2005
(73) Patentinhaber: BASF SE, 67056 Ludwigshafen (DE); Max-Planck-Gesellschaft zur Förderung der Wissenschaften e.V., 80539 München (DE)
(72) Erfinder: BÖHM, Arno, 68305 Mannheim (DE); BLASCHKA, Peter, 67069 Ludwigshafen (DE); HELFER, Willi, 67159 Friedelsheim (DE); HAMMEL, Dirk, 67549 Worms (DE); SCHLICHTING, Peter, 68199 Mannheim (DE); MÜLLEN, Klaus, 50939 Köln (DE)
(74) Vertreter: Schuck, Alexander
(86) Internationale Anmeldenummer: PCT/EP2003/009946
(87) Internationale Veröffentlichungsnummer: WO 2004/029028

(56) Entgegenhaltungen:
- DE-A- 10 038 672
- NAGAO Y ET AL: "SYNTHESIS AND PROPERTIES OF N-ALKYLBROMOPERYLENE-3,4-DICARBOXIMIDES" DYES AND PIGMENTS, ELSEVIER APPLIED SCIENCE PUBLISHERS. BARKING, GB, vol. 16, no. 1, 1991, pages 19-25, XP000208007 ISSN: 0143-7208 cited in the application

## Beschreibung

Die vorliegende Erfindung betrifft neue 9-cyanosubstituierte Perylen-3,4-dicarbonsäuremonoimide der allgemeinen Formel I 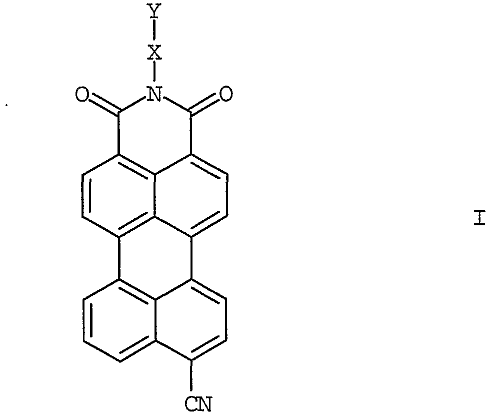 in der die Variablen folgende Bedeutung haben:
- X: eine chemische Bindung;
C₁-C₃₀-Alkylen, dessen Kohlenstoffkette durch eine oder mehrere Gruppierungen -O-, -S-, -NR¹-, -CO- und/oder -SO₂- unterbrochen sein kann und das durch -COOR¹, -SO₃R¹, Cyano, C₁-C₆-Alkoxy, Aryl, das durch C₁-C₁₈-Alkyl und/oder C₁-C₆-Alkoxy substituiert sein kann, und/oder einen über ein Stickstoffatom gebundenen, 5- bis 7-gliedrigen heterocyclischen Rest, der weitere Heteroatome enthalten und aromatisch sein kann, ein- oder mehrfach substituiert sein kann;
C₅-C₈-Cycloalkylen, dessen Kohlenstoffgerüst durch eine oder mehrere Gruppierungen -O-, -S-, -NR¹-, -CO- und/oder -SO₂- unterbrochen und/oder das ein- oder mehrfach durch C₁-C₁₂-Alkyl, -COOR¹, -SO₃R¹, Cyano und/oder C₁-C₆-Alkoxy substituiert sein kann;
Arylen oder Hetarylen, das jeweils ein- oder mehrfach durch C₁-C₁₈-Alkyl, C₁-C₆-Alkoxy, Cyano, -COOR¹, -SO₃R¹, -CONH-R¹ und/oder -NH-COR¹ substituiert sein kann;
C₁-C₂₀-Alkylarylen oder -hetarylen, dessen Alkylengruppe jeweils durch eine oder mehrere Gruppierungen -O-, -S-, -NR¹-, -CO- und/oder -SO₂- unterbrochen sein kann und das jeweils durch -COOR¹, -SO₃R¹, -CONHR¹, -NHCOR¹, Cyano, C₁-C₁₈-Alkyl, C₁-C₆-Alkoxy und/oder einen über ein Stickstoffatom gebundenen, 5- bis 7-gliedrigen heterocyclischen Rest, der weitere Heteroatome enthalten und aromatisch sein kann, ein- oder mehrfach substituiert sein kann;
Aryl- oder Hetaryl-C₁-C₂₀-alkylen, dessen Alkylengruppe jeweils durch eine oder mehrere Gruppierungen -O-, -S-, -NR¹-, -CO- und/oder -SO₂- unterbrochen sein kann und das jeweils durch -COOR¹, -SO₃R¹, -CONHR¹, -NHCOR¹, Cyano, C₁-C₁₈-Alkyl, C₁-C₆-Alkoxy und/oder einen über ein Stickstoffatom gebundenen, 5- bis 7-gliedrigen heterocyclischen Rest, der weitere Heteroatome enthalten und aromatisch sein kann, ein- oder mehrfach substituiert sein kann;
- Y: eine funktionelle Gruppe Y' oder eine polymerisierbare Gruppe P;
oder
- X-Y: gemeinsam einen Rest R;
- Y': Amino, Hydroxy, -COOH, -SO₃H, Chlor oder Brom;
- P: einen Rest der allgemeinen Formel II 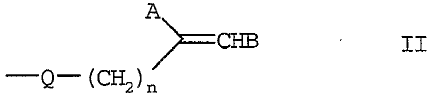
- A, B: unabhängig voneinander Wasserstoff, C₁-C₆-Alkyl, Phenyl oder gemeinsam einen Cyclopenten- oder Cyclohexenring, der die Doppelbindung, an die A und B gebunden sind, enthält;
- Q: eine chemische Bindung;
eine Gruppierung -O-, -NR²-, -S-, -OCO-, -OCOO-, -OCONR³-, -NR³CO-, -NR³COO-, -NR³CONR⁴-, -CO-, -COO-, -CONR³-, -SO₂-O-, -SO₂NR³-, -O-SO₂- oder -NR³SO₂-;
- n: 0, 1, 2 oder 3;
- R: Wasserstoff;
C₁-C₃₀-Alkyl, dessen Kohlenstoffkette durch eine oder mehrere Gruppierungen -O-, -S-, -NR¹-, -CO- und/oder-SO₂- unterbrochen sein kann und das durch Cyano, C₁-C₆-Alkoxy, Aryl, das durch C₁-C₁₈-Alkyl und/oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₆-Alkyl ein- oder mehrfach substituiert sein kann;
Aryl oder Hetaryl, das jeweils durch C₁-C₁₈-Alkyl, C₁-C₆-Alkoxy, Cyano, -CONHR⁵, -NHCOR⁵ und/oder Aryl- oder Hetarylazo, das jeweils durch C₁-C₁₀-Alkyl, C₁-C₆-Alkoxy und/oder Cyano substituiert sein kann, ein- oder mehrfach substituiert sein kann;
- R¹: Wasserstoff oder C₁-C₆-Alkyl;
- R²: Wasserstoff, C₁-C₆-Alkyl, Aryl, Aryl-C₁-C₆-alkyl, C₁-C₆-Alkylcarbonyl, Arylcarbonyl oder Formyl;
- R³,R⁴: unabhängig voneinander Wasserstoff; C₁-C₆-Alkyl; Aryl oder Aryl-C₁-C₆-alkyl, das jeweils durch Hydroxy, Halogen, C₁-C₆-Alkyl und/oder C₁-C₆-Alkoxy substituiert sein kann;
- R⁵: Wasserstoff; C₁-C₁₈-Alkyl; Aryl oder Hetaryl, das jeweils durch C₁-C₆-Alkyl, C₁-C₆-Alkoxy, Halogen, Hydroxy, Carboxy und/oder Cyano substituiert sein kann,
sowie die Herstellung dieser 9-cyanosubstituierten Perylen-3,4-dicarbonsäuremonoimide und ihre Verwendung als Fluoreszenzfarbmittel, zur Einfärbung von hochmolekularen organischen und anorganischen Materialien, insbesondere von Kunststoffen, Lacken, Druckfarben, anorganisch-organischen Compositen und oxidischen Schichtsystemen, als Dispergierhilfsmittel, Pigmentadditive für organische Pigmente und Zwischenprodukte für die Herstellung von Fluoreszenzfarbmitteln und Pigmentadditiven, zur Herstellung von im gelben Bereich des elektromagnetischen Spektrums absorbierenden und/oder emittierenden wäßrigen Polymerisatdispersionen und Ink-Jet-Tinten, als Photoleiter in der Elektrophotographie, als farbgebende bzw. farbkorrigierende Komponente in emissiven und transflektiven Farbfiltern und in retroreflektiven Vorrichtungen, als Emitter in Elektro- und Chemilumineszenzanwendungen, als Aktivkomponenten in der Fluoreszenzkonversion, in. Fluoreszenzsolarkollektoren, in Biolumineszenzarrays sowie in der Photovoltaik und als Laserfarbstoffe.

Weiterhin betrifft die Erfindung das in 9-Position bromierte oder cyanosubstituierte Perylen-3,4-dicarbonsäureanhydrid der Formel III 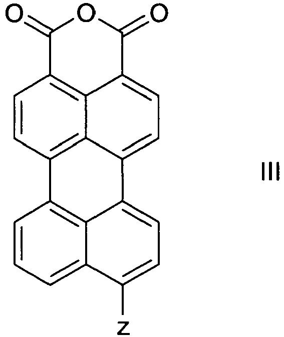 in der Z Brom oder Cyano bedeutet, als Zwischenprodukte bei der Herstellung der erfindungsgemäßen 9-cyanosubstituierten Perylen-3,4-dicarbonsäuremonoimide der Formel I.

N-substituierte Perylen-3,4-dicarbonsäuremonoimide, die im Perylengerüst keine Substituenten tragen, und Verfahren zu ihrer Herstellung sind aus einer Reihe von Veröffentlichungen bekannt (z.B. Shikizai Kyokai Shi 49, 29 (1976), Bull. Chem. Soc. Jpn. 54, 1575 (1981) und EP-A-657 436). In den EP-A-854 144 und WO-A-98/31678 werden zwar im Perylengerüst substituierte Perylen-3,4-dicarbonsäuremonoimide und substituiertes Perylen3,4-dicarbonsäureanhydrid, wobei in der WO-A-98/31678 eine Substitution in 9-Stellung ausgeschlossen ist, beansprucht, explizit offenbart werden jedoch nur die im Perylengerüst unsubstituierten Verbindungen.

Im Perylengerüst substituierte, u.a. auch bromierte, Perylen-3,4-dicarbonsäuremonoimide sind in den WO-A-96/22331 (insbesondere 1,7-disubstituierte Verbindungen) und WO-A-96/22332 (insbesondere 1,6,9-trisubstituierte Verbindungen) beschrieben. Dyes and Pigments 16, 19 (1991), die US-A-5 405 962 und die WO-A-01/16109 betreffen schließlich in 9-Stellung substituierte Perylen-3,4-dicarbonsäuremonoimide, wobei die beiden erstgenannten Veröffentlichungen ausschließlich bromierte Verbindungen beschreiben, cyanosubstituierte Perylen-3,4-dicarbonsäuremonoimide werden in keiner dieser Veröffentlichungen offenbart.

DE 100 38 672 A1 offenbart Perylen-3,4,9-tricarbonsäure-3,4-carbonsäureimide, im Einzelnen 9-Carboxy-[N-(1-hexylheptyl)]-perylen-3,4,9-tricarbonsäure-3,4-dicarbonsäureimid, 9-Carboxy-[N-(1-nonyldecyl)]-perylen-3,4,9-tricarbonsäure-3,4-dicarboximid und 9-Carboxy[N-(2,5-di-tert-butylphenyl)]-perylen-3,4,9-tricarbonsäure-3,4-dicarboximid als Fluoreszenzfarbstoffe.

Alle bekannten Perylen-3,4-dicarbonsäuremonoimide absorbieren und emittieren im orangefarbenen bis blaustichig roten Farbraum. Im gelben Farbraum, also deutlich hypsochromer, absorbierende bzw. emittierende Vertreter dieser Verbindungsklasse sind in der Literatur nicht beschrieben und waren aufgrund der Größe des chromophoren Systems für diese Substanzklasse auch nicht zu erwarten.

Die in den EP-A-854 144 und WO-A-98/31678 beschriebenen Synthesewege zur Herstellung von Perylen-3,4-dicarbonsäureanhydriden (über eine Alkylierungs/Arylierungs-Verseifungs-Sequenz, ausgehend von am Stickstoffatom unsubstituierten Perylen-3,4-dicarbonsäuremonoimiden, bzw. über die direkte partielle baseninduzierte Decarboxylierung von Perylen-3,4:9,10-tetracarbonsäuredianhydriden) sind sehr aufwendig und im technischen Maßstab nicht zu realisieren. Zudem ist 9-Bromperylen-3,4-dicarbonsäureanhydrid aufgrund der Instabilität des Bromsubstituenten unter den angegebenen, basischen Verseifungs- bzw. Decarboxylierungsbedingungen prinzipiell nicht darstellbar.

Die in Dyes and Pigments 16, 19 (1991) und der US-A-5 405 962 zur selektiven Bromierung von N-substituierten Perylen-3,4-dicarbonsäuremonoimiden in der 9-Position in Gegenwart von halogenierten organischen Lösungsmitteln beschriebenen Umsetzungen sind aufgrund der zu geringen Löslichkeit vieler der umzusetzenden Substrate, insbesondere von Perylen-3,4-dicarbonsäureanhydrid selbst, in diesen Solventien bei den maximal zulässigen Reaktionstemperaturen weder universell noch für technische Ansatzgrößen einsetzbar. Zudem sind 9-Bromperylen-3,4-dicarbonsäuremonoimide und ihre Folgeprodukte, die weitere, unter den bei der Bromierung bzw. nukleophilen Substitution herrschenden Reaktionsbedingungen instabile funktionelle Gruppen am Imidstickstoffatom tragen, auf den literaturbekannten Wegen prinzipiell nicht zugänglich.

Der Erfindung lag daher die Aufgabe zugrunde, geeignet substituierte Perylen-3,4-dicarbonsäuremonoimide mit vorteilhaften Anwendungseigenschaften, die insbesondere nicht nur gut in das jeweilige Anwendungsmedium einarbeitbar und an dieses Medium anpaßbar sind, sondern auch kürzerwelliger (hypsochromer) als die bisher bekannten Vertreter dieser Substanzklasse, d.h. im gelben Bereich des elektromagnetischen Spektrums, absorbieren und emittieren, bereitzustellen.

Demgemäß wurden die 9-cyanosubstituierten Perylen-3,4-dicarbonsäuremonoimide der eingangs definierten Formel I gefunden.

Bevorzugte 9-cyanosubstituierte Perylen-3,4-dicarbonsäuremonoimide sind dem Unteranspruch zu entnehmen.

Weiterhin wurde ein Verfahren zur Herstellung von nicht- oder monofunktionellen 9-Cyanoperylen-3,4-dicarbonsäuremonoimiden der allgemeinen Formel Ia 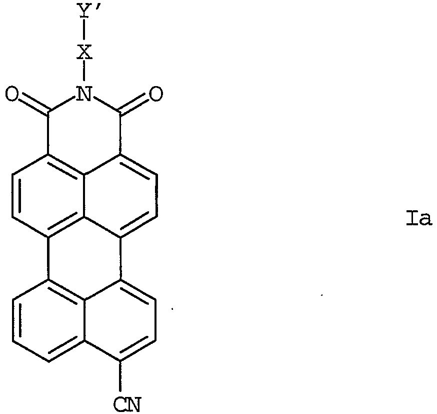 in der X und Y' die eingangs angegebene Bedeutung haben oder X-Y' gemeinsam einen der eingangs definierten Reste R bedeuten, gefunden, welches dadurch gekennzeichnet ist, daß man
a) Perylen-3,4-dicarbonsäureanhydrid in konzentrierter Schwefelsäure oder einer aliphatischen Monocarbonsäure mit elementarem Brom in der 9-Position bromiert,
b) das in Schritt a) erhaltene 9-Bromperylen-3,4-dicarbonsäureanhydrid in einem hochsiedenden inerten Verdünnungsmittel, gegebenenfalls unter Zusatz einer stickstoffbasischen Verbindung oder eines Stickstoffheterocyclus als Katalysator, mit Kupfer(I)cyanid im Überschuß umsetzt und
c) das in Schritt b) erhaltene 9-Cyanoperylen-3,4-dicarbonsäureanhydrid in Wasser oder einem inerten organischen Lösungsmittel, gegebenenfalls unter Zusatz eines Imidierungskatalysators, mit einem primären Amin der allgemeinen Formel IV

   Y'-X-NH₂ IV

   zu dem gewünschten 9-Cyanoperylen-3,4-dicarbonsäuremonoimid der Formel Ia umsetzt.

Außerdem wurde ein Verfahren zur Herstellung von eine radikalisch polymerisierbare Gruppe enthaltenden 9-Cyanoperylen-3,4-dicarbonsäuremonoimiden der allgemeinen Formel Ib 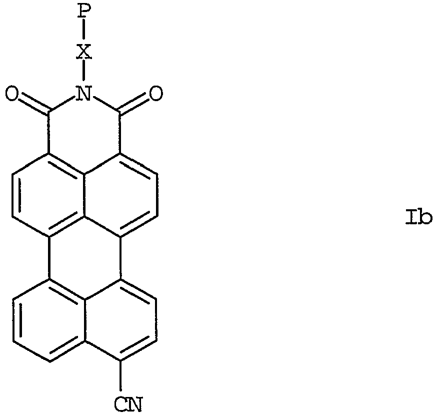 in der X die eingangs angegebene Bedeutung hat und P einen der eingangs definierten Reste der Formel II, in der Q für -OCO- oder -NHCO- steht, bedeutet, gefunden, welches dadurch gekennzeichnet ist, daß man ein Perylen-3,4-dicarbonsäuremonoimid der Formel Ia, in der Y' Amino oder Hydroxy bedeutet, in einem inerten aprotischen Verdünnungsmittel unter Zusatz einer Stickstoffbase mit einem Carbonsäurechlorid der allgemeinen Formel V 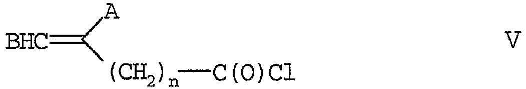 in der die Variablen die eingangs angegebene Bedeutung haben, umsetzt.

Schließlich wurde ein Verfahren zur Herstellung von 9-Cyanoperylendicarbonsäuremonoimiden der allgemeinen Formel Ic 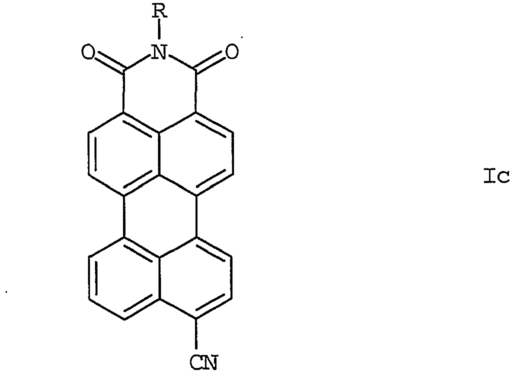 in der R die eingangs angegebene Bedeutung hat, gefunden, welches dadurch gekennzeichnet ist, daß man ein 9-Bromperylen-3,4-dicarbonsäuremonoimid der allgemeinen Formel VI 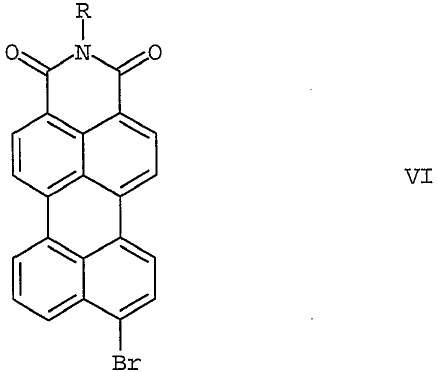 durch Umsetzung mit Kupfer(I)cyanid in Substanz oder in einem hochsiedenden inerten Verdünnungsmittel, gegebenenfalls unter Zusatz einer stickstoffbasischen Verbindung oder eines Stickstoffheterocyclus als Katalysator, in das gewünschte 9-Cyanoperylen-3,4-dicarbonsäuremonoimid der Formel Ic überführt.

Nicht zuletzt wurden in 9-Position substituierte Perylen-3,4-dicarbonsäureanhydride der allgemeinen Formel III 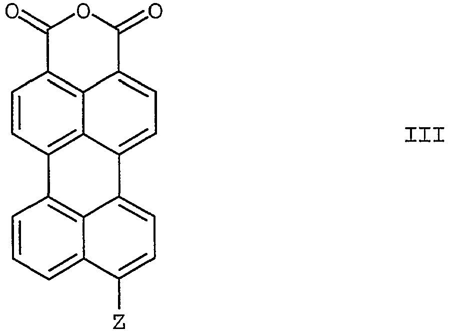 in der Z Brom oder Cyano bedeutet, sowie die Verfahren zur Herstellung dieser Perylen-3,4-dicarbonsäureanhydride gefunden, welche dadurch gekennzeichet sind, daß man Perylen-3,4-dicarbonsäureanhydrid in konzentrierter Schwefelsäure oder einer aliphatischen Monocarbonsäure mit elementarem Brom selektiv in der 9-Position bromiert bzw. das dabei erhaltene 9-Bromperylen-3,4-dicarbonsäureanhydrid IIIa in einem hochsiedenden inerten Verdünnungsmittel, gegebenenfalls unter Zusatz einer stickstoffbasischen Verbindung oder eines Stickstoffheterocyclus als Katalysator, mit Kupfer(I)cyanid im Überschuß umsetzt.

Nicht zuletzt wurde die Verwendung der 9-cyanosubstituierten Perylen-3,4-dicarbonsäuremonoimide I als Fluoreszenzfarbmittel, zur Einfärbung von hochmolekularen organischen und anorganischen Materialien, insbesondere von Kunststoffen, Lacken, Druckfarben, anorganisch-organischen Compositen und oxidischen Schichtsystemen, als Dispergierhilfsmittel, Pigmentadditive für organische Pigmente und Zwischenprodukte für die Herstellung von Fluoreszenzfarbmitteln und Pigmentadditiven, zur Herstellung von im gelben Bereich des elektromagnetischen Spektrums absorbierenden und/ oder emittierenden wäßrigen Polymerisatdispersionen und Ink-Jet-Tinten, als Photoleiter in der Elektrophotographie, als farbgebende bzw. farbkorrigierende Komponente in emissiven und transflektiven Farbfiltern und in retroreflektiven Vorrichtungen, als Emitter in Elektro- und Chemilumineszenzanwendungen, als Aktivkomponenten in der Fluoreszenzkonversion, in Fluoreszenzsolarkollektoren, in Biolumineszenzarrays sowie der Photovoltaik und als Laserfarbstoffe gefunden.

Alle in den Formeln I bis VI auftretenden Alkylgruppen können geradkettig oder verzweigt sein. Wenn die Alkylgruppen substituiert sind, tragen sie in der Regel 1 oder 2 Substituenten.

Aromatische Reste, die substituiert sind, können im allgemeinen bis zu 3, bevorzugt 1 oder 2, der genannten Substituenten aufweisen.

Als Beispiele für geeignete Reste R, R¹, R², R³, R⁴, R⁵, X, Y, Q, A und B (bzw. für deren Substituenten) seien im einzelnen genannt:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen);
Methylen, Ethylen, Propylen, Isopropylen, Butylen, Isobutylen, tert.-Butylen, Pentylen, Isopentylen, Neopentylen, tert.-Pentylen, Hexylen, 2-Methylpentylen, Heptylen, 1-Ethylpentylen, Octylen, 2-Ethylhexylen, Isooctylen, Nonylen, Isononylen, Decylen, Isodecylen, Undecylen, Dodecylen, Tridecylen, Isotridecylen, Tetradecylen, Pentadecylen, Hexadecylen, Heptadecylen, Octadecylen, Nonadecylen und Eicosylen (die obigen Bezeichnungen Isooctylen, Isononylen, Isodecylen und Isotridecylen sind Trivialbezeichnungen und stammen von den nach der Oxosynthese erhaltenen Alkoholen);
Methoxymethyl, 2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2- und 3-Propoxypropyl, 2- und 3-Butoxypropyl, 2-und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
2-Oxapropylen, 2- und 3-Oxabutylen, 2-, 3- und 4-Oxapentylen, 2-, 3-, 4- und 5-Oxahexylen, 2- und 3-Oxa-4-methylpentylen,2- und 4-Oxa-3-methylpentylen, 2-, 3-, 4-, 5- und 6-Oxaheptylen, 2-, 3-, 4-, 5-, 6- und 7-Oxaoctylen, 2-, 3-, 4-, 5-, 6-, 7- und 8-Oxanonylen, 3,6-Dioxaheptylen, 3,6-Dioxaoctylen, 4,8-Dioxanonylen, 3,7-Dioxaoctylen, 3,7-Dioxanonylen, 4,7-Dioxaoctylen, 4,7-Dioxanonylen, 4,8-Dioxadecylen, 3,6,9-Trioxadecylen, 3,6,9-Trioxaundecylen, 3,6,9-Trioxadodecylen, 3,6,9,12-Tetraoxatridecylen und 3,6,9,12-Tetraoxatetradecylen;
Methylthiomethyl, 2-Methylthioethyl, 2-Ethylthioethyl, 2-Propylthioethyl, 2-Isopropylthioethyl, 2-Butylthioethyl, 2- und 3-Methylthiopropyl, 2- und 3-Ethylthiopropyl, 2- und 3-Propylthiopropyl, 2- und 3-Butylthiopropyl, 2- und 4-Methylthiobutyl, 2- und 4-Ethylthiobutyl, 2- und 4-Propylthiobutyl, 3,6-Dithiaheptyl, 3,6-Dithiaoctyl, 4,8-Dithianonyl, 3,7-Dithiaoctyl, 3,7-Dithianonyl, 2- und 4-Butylthiobutyl, 4,8-Dithiadecyl, 3,6,9-Trithiadecyl, 3,6,9-Trithiaundecyl, 3,6,9-Trithiadodecyl, 3,6,9,12-Tetrathiatridecyl und 3,6,9,12-Tetrathiatetradecyl;
2-Thiapropylen, 2- und 3-Thiabutylen, 2-, 3- und 4-Thiapentylen, 2-, 3-, 4- und 5-Thiahexylen, 2- und 3-Thia-4-methylpentylen,2-und 4-Thia-3-methylpentylen, 2-, 3-, 4-, 5- und 6-Thiaheptylen, 2-, 3-, 4-, 5-, 6- und 7-Thiaoctylen, 2-, 3-, 4-, 5-, 6-, 7- und 8-Thianonylen, 3,6-Dithiaheptylen, 3,6-Dithiaoctylen, 4,8-Dithianonylen, 3,7-Dithiaoctylen, 3,7-Dithianonylen, 4,7-Dithiaoctylen, 4,7-Dithianonylen, 4,8-Dithiadecylen, 3,6,9-Trithiadecylen, 3,6,9-Trithiaundecylen, 3,6,9-Trithiadodecylen, 3,6,9,12-Tetrathiatridecylen und 3,6,9,12-Tetrathiatetradecylen;
2-Monomethyl- und 2-Monoethylaminoethyl, 2-Dimethylaminoethyl, 2-und 3- Dimethylaminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Diazaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
N-Methyl-, N-Ethyl-, N-Propyl-, N-Isopropyl- und N-Butyl-2-azapropylen, N-Methyl-, N-Ethyl-, N-Propyl-, N-Isopropyl- und N-Butyl-2-azabutylen, N-Methyl-, N-Ethyl-, N-Propyl-, N-Isopropyl- und N-Butyl-3-azabutylen, N-Methyl-, N-Ethyl-, N-Propyl-, N-Isopropyl- und N-Butyl-2-azapentylen, N-Methyl-, N-Ethyl-, N-Propyl-, N-Isopropyl- und N-Butyl-3-azapentylen, N-Methyl-, N-Ethyl-, N-Propyl-, N-Isopropyl- und N-Butyl-4-azapentylen, N-Methyl-, N-Ethyl-, N-Propyl-, N-Isopropyl- und N-Butyl-2-azahexylen, N-Methyl-, N-Ethyl-, N-Propyl-, N-Isopropyl- und N-Butyl-3-azahexylen, N-Methyl-, N-Ethyl-, N-Propyl-, N-Isopropyl- und N-Butyl-4-azahexylen, N-Methyl-, N-Ethyl-, N-Propyl-, N-Isopropyl- und N-Butyl-5-azahexylen, 3-Methyl-3,6-diazaheptylen, 6-Methyl-3,6-diazaheptylen, 3,6-Dimethyl-3,6-diazaheptylen, 3,6-Diazaoctylen, 3,6-Dimethyl-3,6-diazaoctylen, 3-Methyl-3,6,9-triazadecylen, 6-Methyl-3,6,9-triazadecylen, 9-Methyl-3,6,9-triazadecylen, 3,6,9-Trimethyl-3,6,9-triazadecylen, 3,6,9-Triazaundecylen, 3,6,9-Trimethyl-3,6,9-triazaundecylen, 3-Methyl-3,6,9,12-tetraazatridecylen, 6-Methyl-3,6,9,12-tetraazatridecylen, 9-Methyl-3,6,9,12-tetraazatridecylen, 12-Methyl-3,6,9,12-tetraazatridecylen und 3,6,9,12-Tetramethyl-3,6,9,12-tetraazatridecylen;
Propan-2-on-1-yl, Butan-3-on-1-yl, Butan-3-on-2-yl und 2-Ethylpentan-3-on-1-yl;
Propylen-2-on, Butylen-2-on, Butylen-3-on und 2-Ethylpentylen-3-on, 2-Ethylpentylen-4-on, 3-Ethylpentylen-2-on und 3-Ethylpentylen-4-on;
2-Methylsulfonylethyl, 2-Ethylsulfonylethyl, 2-Propylsulfonylethyl, 2-Isopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylpropyl, 2- und 4-Methylsulfonylbutyl, 2- und 4-Ethylsulfonylbutyl, 2- und 4-Propylsulfonylbutyl und 4-Butylsulfonylbutyl;
Methylen-, Ethylen-, Propylen-, Isopropylen-, Butylen-, Isobutylen-, sec.-Butylen-, tert.-Butylen- und Pentylensulfonylmethylen, Methylen-, Ethylen-, Propylen-, Isopropylen-, Butylen-, Isobutylen-, sec.-Butylen-, tert.-Butylen- und Pentylensulfonylethylen, Methylen-, Ethylen-, Propylen-, Isopropylen-, Butylen-, Isobutylen-, sec.-Butylen-, tert.-Butylen- und Pentylensulfonylpropylen, Methylen-, Ethylen-, Propylen-, Isopropylen-, Butylen-, Isobutylen-, sec.-Butylen-, tert.-Butylen- und Pentylensulfonylisopropylen, Methylen-, Ethylen-, Propylen-, Isopropylen-, Butylen-, Isobutylen-, sec.-Butylen-, tert.-Butylen- und Pentylensulfonylbutylen, Methylen-, Ethylen-, Propylen-, Isopropylen-, Butylen-, Isobutylen-, sec.-Butylen-, tert.-Butylen- und Pentylensulfonylisobutylen, Methylen-, Ethylen-, Propylen-, Isopropylen-, Butylen-, Isobutylen-, sec.-Butylen-, tert.-Butylen- und Pentylensulfonyl-sec.-butylen, Methylen-, Ethylen-, Propylen-, Isopropylen-, Butylen-, Isobutylen-, sec.-Butylen-, tert.-Butylen- und Pentylensulfonyl-tert.-butylen, Methylen-, Ethylen-, Propylen-, Isopropylen-, Butylen-, Isobutylen-, sec.-Butylen-, tert.-Butylen- und Pentylensulfonylpentylen, 1,2-Bis(methylensulfonyl)ethan, 1,2- und 1,3-Bis(methylensulfonyl)propan, 1,2-, 1,3- und 1,4-Bis(methylensulfonyl)butan, 1,2-, 1,3-, 1,4- und 1,5-Bis(methylensulfonyl)pentan, 1,2-Bis(ethylensulfonyl)ethan, 1,2- und 1,3-Bis(ethylensulfonyl)propan, 1,2-, 1,3- und 1,4-Bis(ethylensulfonyl)butan, 1,2-, 1,3-, 1,4- und 1,5-Bis(ethylensulfonyl)pentan, 1,2-Bis(propylensulfonyl)ethan, 1,2- und 1,3-Bis(propylensulfonyl)propan, 1,2-, 1,3- und 1,4-Bis(propylensulfonyl)butan, 1,2-, 1,3-, 1,4- und 1,5-Bis(propylensulfonyl)pentan;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxytetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;
2-Hydroxyethyl, 3-Hydroxypropyl, 1-Hydroxyprop-2-yl, 2- und 4-Hydroxybutyl, 1-Hydroxybut-2-yl und 8-Hydroxy-4-oxaoctyl;
Cyanomethyl, 2-Cyanoethyl, 3-Cyanopropyl, 2-Methyl-3-ethyl-3-cyanopropyl, 7-Cyano-7-ethylheptyl und 4,7-Dimethyl-7-cyanoheptyl;
Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;
Carbamoyl, Methylaminocarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl, Decylaminocarbonyl und Phenylaminocarbonyl;
Formylamino, Acetylamino, Propionylamino und Benzoylamino;
Chlor, Brom und Iod;
Phenyl, 1- und 2-Naphthyl, 2- und 3-Pyrryl, 2-, 3- und 4-Pyridyl, 2-, 4- und 5-Pyrimidyl, 2-Pyrazyl, 3-, 4- und 5-Pyrazolyl, 2-, 4-und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothiazolyl, 5-Benzothiadiazolyl, 2- und 5-Benzimidazolyl und 1- und 5-Isochinolyl;
1,2-, 1,3- und 1,4-Phenylen, 1,2-, 2,3-, 1,4-, 1,5-, 2,6- und 1,8-Naphthylen, 2,5- und 3,4-Pyrrylen, 2,3-, 2,4-, 2,5-, 2,6- und 3,5-Pyridylen, 2,4- und 2,5-Pyrimidylen, 2,3-, 2,5- und 2,6-Pyrazylen, 3,5-Pyrazolylen, 1,2-, 1,4-, 1,5-, 2,4- und 2,5-Imidazolylen, 2,4- und 2,5-Thiazolylen, 3,5- und 3,6-(1,2,4-Triazylen), 2,4-(1,3,5-Triazylen), 3,5-, 3,6-, 3,8- und 5,8-Chinaldylen, 3,5-, 3,6-, 3,8- und 5,8-Chinolinylen, 2,4- und 2,5-Benzimidazolylen und 1,3-, 1,4-, 1,5-und 1,6-Isochinolylen;
2-, 3- und 4-Methylphenyl, 2,3-, 2,4-, 2,5-, 3,5- und 2,6-Dimethylphenyl, 2,4,6-Trimethylphenyl, 2-, 3- und 4-Ethylphenyl, 2,3-, 2,4-, 2,5-, 3,5- und 2,6-Diethylphenyl, 2,4,6-Triethylphenyl, 2-, 3- und 4-Propylphenyl, 2,3-, 2,4-, 2,5-, 3,5- und 2,6-Dipropylphenyl, 2,4,6-Tripropylphenyl, 2-, 3- und 4-Isopropylphenyl, 2,3-, 2,4-, 2,5-, 3,5- und 2,6-Diisopropylphenyl, 2,4,6-Triisopropylphenyl, 2-, 3- und 4-Butylphenyl, 2,3-, 2,4-, 2,5-, 3,5- und 2,6-Dibutylphenyl, 2,4,6-Tributylphenyl, 2-, 3- und 4-Isobutylphenyl, 2,3-, 2,4-, 2,5-, 3,5- und 2,6-Diisobutylphenyl, 2,4,6-Triisobutylphenyl, 2-, 3- und 4-sec.-Butylphenyl, 2,3-, 2,4-, 2,5-, 3,5- und 2,6-Di-sec.-butylphenyl und 2,4,6-Tri-sec.-butylphenyl, 2-, 3- und 4-tert.-Butylphenyl, 2,3-, 2,4-, 2,5-, 3,5- und 2,6-Di-tert.-butylphenyl, 2,4,6-Tri-tert.-butylphenyl; 2-, 3- und 4-Methoxyphenyl, 2,3-, 2,4-, 2,5-, 3,5- und 2,6-Dimethoxyphenyl, 2,4,6-Trimethoxyphenyl, 2-, 3- und 4-Ethoxyphenyl, 2,3-, 2,4-, 2,5-, 3,5- und 2,6-Diethoxyphenyl, 2,4,6-Triethoxyphenyl, 2-, 3- und 4-Propoxyphenyl, 2,3-, 2,4-, 2,5-, 3,5- und 2,6-Dipropoxyphenyl, 2-, 3- und 4-Isopropoxyphenyl, 2,3-, 2,4-, 2,5-, 3,5- und 2,6-Diisopropoxyphenyl und 2-, 3- und 4-Butoxyphenyl; 2-, 3- und 4-Chlorphenyl, und 2,3-, 2,4-, 2,5-, 3,5- und 2,6-Dichlorphenyl; 2-, 3- und 4-Hydroxyphenyl und 2,3-, 2,4-, 2,5-, 3,5- und 2,6-Dihydroxyphenyl; 2-, 3- und 4-Cyano-phenyl; 3- und 4-Carboxyphenyl; 3- und 4-Carboxamidophenyl, 3- und 4-N-Methylcarboxamidophenyl und 3- und 4-N-Ethylcarboxamidophenyl; 3- und 4-Acetylaminophenyl, 3- und 4-Propionylaminophenyl und 3- und 4-Butyrylaminophenyl; 3- und 4-N-Phenylaminophenyl, 3- und 4-N-(o-Tolyl)aminophenyl, 3- und 4-N-(m-Tolyl)aminophenyl und 3- und 4-N-(p-Tolyl)aminophenyl; 3- und 4-(2-Pyridyl)aminophenyl, 3- und 4-(3-Pyridyl)aminophenyl, 3- und 4-(4-Pyridyl)aminophenyl, 3- und 4-(2-Pyrimidyl)aminophenyl und 4-(4-Pyrimidyl)aminophenyl;
2-Methyl-1,3-, -1,4-, -1,5- und -1,6-phenylen, 2,3-Dimethyl-1,4-, -1,5- und -1,6-phenylen, 2,4-Dimethyl-1,3-, -1,5- und -1,6-phenylen, 2,5-Dimethyl-1,3-, -1,4- und -1,6-phenylen, 2,4,5-Trimethyl-1,3- und -1,6-phenylen, 2,4,6-Tri-methyl-1,3-phenyleh, 2-Ethyl-1,3-, -1,4-, -1,5- und -1,6-phenylen, 2,3-Diethyl-1,4-, -1,5- und -1,6-phenylen, 2,4-Diethyl-1,3-, -1,5- und -1,6-phenylen, 2,5-Diethyl-1,3-, -1,4- und -1,6-phenylen, 2,4,5-Triethyl-1,3- und -1,6-phenylen, 2,4,6-Triethyl-1,3-phenylen, 2-Propyl-1,3-, -1,4-, -1,5- und -1,6-phenylen, 2,3-Dipropyl-1,4-, -1,5- und -1,6-phenylen, 2,4-Dipropyl-1,3-, -1,5- und -1,6-phenylen, 2,5-Dipropyl-1,3-, -1,4- und -1,6-phenylen, 2,4,5-Tripropyl-1,3- und -1,6-phenylen, 2,4,6-Tripropyl-1,3-phenylen, 2-Isopropyl-1,3-, -1,4-, -1,5- und -1,6-phenylen, 2,3-Diisopropyl-1,4-, -1,5- und -1,6-phenylen, 2,4-Diisopropyl-1,3-, -1,5- und -1,6-phenylen, 2,5-Diisopropyl-1,3-, -1,4- und -1,6-phenylen, 2,4,5-Triisopropyl-1,3- und -1,6-phenylen, 2,4,6-Triisopropyl-1,3-phenylen, 2-Butyl-1,3-, -1,4-, -1,5- und -1,6-phenylen, 2,3-Dibutyl-1,4-, -1,5- und -1,6-phenylen, 2,4-Dibutyl-1,3-, -1,5- und -1,6-phenylen, 2,5-Dibutyl-1,3-, -1,4- und -1,6-phenylen, 2,4,5-Tributyl-1,3- und -1,6-phenylen, 2,4,6-Tributyl-1,3-phenylen, 2-Isobutyl-1,3-, -1,4-, -1,5- und -1,6-phenylen, 2,3-Diisobutyl-1,4-, -1,5- und -1,6-phenylen, 2,4-Diisobutyl-1,3-, -1,5- und -1,6-phenylen, 2,5-Diisobutyl-1,3-, -1,4- und -1,6-phenylen, 2,4,5-Triiso-butyl-1,3- und -1,6-phenylen, 2,4,6-Triisobutyl-1,3-phenylen, 2-sec.-Butyl-1,3-, -1,4-, -1,5- und -1,6-phenylen, 2,3-Di-sec.-butyl-1,4-, -1,5- und -1,6-phenylen, 2,4-Di-sec.-butyl-1,3-, -1,5- und -1,6-phenylen, 2,5-Di-sec.-butyl-1,3-, -1,4- und -1,6-phenylen, 2,4,5-Tri-sec.-butyl-1,3- und -1,6-phenylen, 2,4,6-Tri-sec.-butyl-1,3-phenylen, 2-tert.-Butyl-1,3-, -1,4-, -1,5- und -1,6-phenylen, 2,3-Di-tert.-butyl-1,4-, -1,5- und -1,6-phenylen, 2,4-Di-tert.-butyl-1,3-, -1,5- und -1,6-phenylen, 2,5-Di-tert.-butyl-1,3-, -1,4- und -1,6-phenylen, 2,4,5-Tri-tert.-butyl-1,3- und -1,6-phenylen, 2,4,6-Tri-tert.-butyl-1,3-phenylen; 2-Methoxy-1,3-, -1,4-, -1,5- und -1,6-phenylen, 2,3-Dimethoxy-1,4-, -1,5- und -1,6-phenylen, 2,4-Dimethoxy-1,3-, -1,5- und -1,6-phenylen, 2,5-Dimethoxy-1,3-, -1,4- und -1,6-phenylen, 2,4,5-Trimethoxy-1,3- und -1,6-phenylen, 2,4,6-Trimethoxy-1,3-phenylen, 2-Ethoxy-1,3-, -1,4-, -1,5- und -1,6-phenylen, 2,3-Diethoxy-1,4-, -1,5- und -1,6-phenylen, 2,4-Diethoxy-1,3-, -1,5- und -1,6-phenylen, 2,5-Diethoxy-1,3-, -1,4- und -1,6-phenylen, 2,4,5-Triethoxy-1,3- und -1,6-phenylen, 2,4,6-Triethoxy-1,3-phenylen, 2-Propoxy-1,3-, -1,4-, -1,5- und -1,6-phenylen, 2,3-Dipropoxy-1,4-, -1,5- und -1,6-phenylen, 2,4-Dipropoxy-1,3-, -1,5- und -1,6-phenylen, 2,5-Dipropoxy-1,3-, -1,4- und -1,6-phenylen, 2,4,5-Tripropoxy-1,3- und -1,6-phenylen, 2,4,6-Tripropoxy-1,3-phenylen, 2-Isopropoxy-1,3-, -1,4-, -1,5- und -1,6-phenylen, 2,3-Diisopropoxy-1,4-, -1,5- und -1,6-phenylen, 2,4-Diisopropoxy-1,3-, -1,5- und -1,6-phenylen, 2,5-Diisopropoxy-1,3-, -1,4- und -1,6-phenylen, 2,4,5-Triiso-propoxy-1,3- und -1,6-phenylen, 2,4,6-Triisopropoxy-1,3-phenylen, 2-Butoxy-1,3-, -1,4-, -1,5- und -1,6-phenylen, 2,3-Dibutoxy-1,4-, -1,5- und -1,6-phenylen, 2,4-Dibutoxy-1,3-, -1,5- und -1,6-phenylen, 2,5-Dibutoxy-1,3-, -1,4- und -1,6-phenylen, 2,4,5-Tributoxy-1,3- und -1,6-phenylen, 2,4,6-Tri-butoxy-1,3-phenylen; 2-Chlor-1,3-, -1,4-, -1,5- und -1,6-phenylen, 2,3-Dichlor-1,4-, -1,5- und -1,6-phenylen, 2,4-Dichlor-1,3-, -1,5- und -1,6-phenylen, 2,5-Dichlor-1,3-, -1,4- und -1,6-phenylen; 2-Hydroxy-1,3-, -1,4-, -1,5- und -1,6-phenylen, 2,3-Dihydroxy-1,4-, -1,5- und -1,6-phenylen, 2,4-Dihydroxy-1,3-, -1,5- und -1,6-phenylen, 2,5-Dihydroxy-1,3-, -1,4- und -1,6-phenylen; 2-Cyano-1,3-, -1,4-, -1,5- und -1,6-phenylen, 2,3-Dicyano-1,4-, -1,5- und -1,6-phenylen, 2,4-Dicyano-1,3-, -1,5- und -1,6-phenylen, 2,5-Dicyano-1,3-, -1,4- und -1,6-phenylen;
4-Phenylazophenyl, 4-(1-Naphthylazo)phenyl, 4-(2-Naphthylazo)phenyl, 4-(4-Naphthylazo)phenyl, 4-(2-Pyridylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo)phenyl;
Cyclopentyl, 2- und 3-Methylcyclopentyl, 2- und 3-Ethylcyclopentyl, Cyclohexyl, 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methylcycloheptyl, 2-, 3- und 4-Ethylcycloheptyl, 3- und 4-Propylcycloheptyl, 3- und 4-Isopropylcycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethylcyclooctyl, 3-, 4- und 5-Propylcyclooctyl; 2-Dioxanyl, 4-Morpholinyl, 2- und 3-Tetrahydrofuryl, 1-, 2- und 3-Pyrrolidinyl und 1-, 2-, 3- und 4-Piperidyl;
1,2- und 1,3-Cyclopentylen, 1,3-, 1,4- und 1,5-(2-Methyl)cyclopentylen, 1,3-, 1,4- und 1,5-(2-Ethyl)cyclopentylen, 1,2-, 1,3- und 1,4-Cyclohexylen, 1,3-, 1,4-, 1,5- und 1,6-(2-Methyl)cyclohexylen, 1,3-, 1,4-, 1,5- und 1,6-(2-Ethyl)cyclohexylen, 1,3-, 1,4-, 1,5- und 1,6-(2-Propyl)cyclohexylen, 1,3-, 1,4-, 1,5- und 1,6-(2-Isopropyl)cyclohexylen, 1,3-, 1,4-, 1,5- und 1,6-(2-Butyl)cyclohexylen, 1,3-, 1,4-, 1,5- und 1,6-(2-sec.-Butyl)cyclohexylen, 1,3-, 1,4-, 1,5- und 1,6-(2-tert.-Butyl)cyclohexylen, 1,2-, 1,3-, 1,4-Cycloheptylen, 1,3-, 1,4-, 1,5-, 1,6- und 1,7-(2-Methyl)cycloheptylen, 1,3-, 1,4-, 1,5-, 1,6- und 1,7-(2-Ethyl)cycloheptylen, 1,3-, 1,4-, 1,5-, 1,6- und 1,7-(2-Propyl)cycloheptylen, 1,3-, 1,4-, 1,5-, 1,6- und 1,7-(2-Isopropyl)cycloheptylen, 1,3-, 1,4-, 1,5-, 1,6- und 1,7-(2-Butyl)cycloheptylen, 1,3-, 1,4-, 1,5-, 1,6- und 1,7-(2-sec.-Butyl)cycloheptylen, 1,3-, 1,4-, 1,5-, 1,6- und 1,7-(2-tert.-Butyl)cycloheptylen, 1,2-, 1,3-, 1,4- und 1,5-Cyclooctylen, 1,3-, 1,4-, 1,5-, 1,6-, 1,7- und 1,8-(2-Methyl)cyclooctylen, 1,3-, 1,4-, 1,5-, 1,6-, 1,7- und 1,8-(2-Ethyl)cyclooctylen, 1,3-, 1,4-, 1,5-, 1,6-, 1,7- und 1,8-(2-Propyl)cyclooctylen; 2,3-, 2,5- und 2,6-(1,4-Dioxanylen), 2,4- und 3,4-Morpholinylen, 2,5- und 3,4-Tetrahydrofurylen, 1,2-, 1,3-, 2,5- und 3,4-Pyrrolidinylen und 1,2-, 1,3-, 1,4-, 2,3- und 2,6-Piperidylen;
Dimethylamino, Diethylamino, Dipropylamino, Diisopropylamino, Dibutylamino, Diisobutylamino, Di-tert.-butylamino, Dipentylamino, Dihexylamino, Diphenylamino, Di-o-tolylamino, Di-m-tolylamino, Di-p-tolylamino, Di(4-cyanophenyl)amino;
Methylen-, Ethylen-, Propylen-, Isopropylen-, Butylen-, Isobutylen-, sec.-Butylen- und tert.-Butylen-1,2-, -1,3- und -1,4-phenylen, Methylen-, Ethylen-, Propylen-, Isopropylen-, Butylen-, Isobutylen-, sec.-Butylen- und tert.-Butylen-1,3-, -1,4-, -1,5- und -1,6-(2-methyl)phenylen, Methylen-, Ethylen-, Propylen-, Isopropylen-, Butylen-, Isobutylen-, sec.-Butylen- und tert.-Butylen-1,4-, -1,5- und -1,6-(2,3-dimethyl)phenylen, Methylen-, Ethylen-, Propylen-, Isopropylen-, Butylen-, Isobutylen-, sec.-Butylen- und tert.-Butylen-1,3-, -1,5- und -1,6-(2,4-dimethyl)phenylen, Methylen-, Ethylen-, Propylen-, Isopropylen-, Butylen-, Isobutylen-, sec.-Butylen- und tert.-Butylen-1,3-, -1,4- und -1,6-(2,5-dimethyl)phenylen, Methylen-, Ethylen-, Propylen-, Isopropylen-, Butylen-, Isobutylen-, sec.-Butylen- und tert.-Butylen-1,3- und -1,6-(2,4,5-trimethyl)phenylen, Methylen-, Ethylen-, Propylen-, Isopropylen-, Butylen-, Isobutylen-, sec.-Butylen- und tert.-Butylen-1,3-(2,4,6-trimethyl)phenylen, Methylen-, Ethylen-, Propylen-, Isopropylen-, Butylen-, Isobutylen-, sec.-Butylen- und tert.-Butylen-1,3-, -1,4-, -1,5- und -1,6-(2-ethyl)phenylen, Methylen-, Ethylen-, Propylen-, Isopropylen-, Butylen-, Isobutylen-, sec.-Butylen- und tert.-Butylen-1,4-, -1,5- und -1,6-(2,3-diethyl)phenylen, Methylen-, Ethylen-, Propylen-, Isopropylen-, Butylen-, Isobutylen-, sec.-Butylen- und tert.-Butylen-1,3-, -1,5- und -1,6-(2,4-diethyl)phenylen, Methylen-, Ethylen-, Propylen-, Isopropylen-, Butylen-, Isobutylen-, sec.-Butylen- und tert.-Butylen-1,3-, -1,4- und -1,6-(2,5-diethyl)phenylen, Methylen-, Ethylen-, Propylen-, Isopropylen-, Butylen-, Isobutylen-, sec.-Butylen- und tert.-Butylen-1,3- und -1,6-(2,4,5-triethyl)phenylen, Methylen-, Ethylen-, Propylen-, Isopropylen-, Butylen-, Isobutylen-, sec.-Butylen- und tert.-Butylen-1,3-(2,4,6-triethyl)phenylen, Methylen-, Ethylen-, Propylen-, Isopropylen-, Butylen-, Isobutylen-, sec.-Butylen- und tert.-Butylen-1,3-, -1,4-, -1,5- und -1,6-(2-propyl)phenylen, Methylen-, Ethylen-, Propylen-, Isopropylen-, Butylen-, Isobutylen-, sec.-Butylen- und tert.-Butylen-1,4-, -1,5- und -1,6-(2,3-dipropyl)phenylen, Methylen-, Ethylen-, Propylen-, Isopropylen-, Butylen-, Isobutylen-, sec.-Butylen- und tert.-Butylen-1,3-, -1,5- und -1,6-(2,4-dipropyl)phenylen, Methylen-, Ethylen-, Propylen-, Isopropylen-, Butylen-, Isobutylen-, sec.-Butylen- und tert.-Butylen-1,3-, -1,4- und -1,6-(2,5-dipropyl)phenylen, Methylen-, Ethylen-, Propylen-, Isopropylen-, Butylen-, Isobutylen-, sec.-Butylen- und tert.-Butylen-1,3- und -1,6-(2,4,5-tripropyl)phenylen, Methylen-, Ethylen-, Propylen-, Isopropylen-, Butylen-, Isobutylen-, sec.-Butylen- und tert.-Butylen-1,3-(2,4,6-tripropyl)phenylen, Methylen-, Ethylen-, Propylen-, Isopropylen-, Butylen-, Isobutylen-, sec.-Butylen- und tert.-Butylen-1,3-, -1,4-, -1,5- und -1,6-(2-isopropyl)phenylen, Methylen-, Ethylen-, Propylen-, Isopropylen-, Butylen-, Isobutylen-, sec.-Butylen- und tert.-Butylen-1,4-, -1,5- und -1,6-(2,3-diisopropyl)phenylen, Methylen-, Ethylen-, Propylen-, Isopropylen-, Butylen-, Isobutylen-, sec.-Butylen- und tert.-Butylen-1,3-, -1,5- und -1,6-(2,4-diisopropyl)phenylen, Methylen-, Ethylen-, Propylen-, Isopropylen-, Butylen-, Isobutylen-, sec.-Butylen- und tert.-Butylen-1,3-, -1,4- und -1,6-(2,5-diisopropyl)phenylen, Methylen-, Ethylen-, Propylen-, Isopropylen-, Butylen-, Isobutylen-, sec.-Butylen- und tert.-Butylen-1,3- und -1,6-(2,4,5-triisopropyl)phenylen, Methylen-, Ethylen-, Propylen-, Isopropylen-, Butylen-, Isobutylen-, sec.-Butylen- und tert.-Butylen-1,3-(2,4,6-triisopropyl)phenylen, Methylen-, Ethylen-, Propylen-, Isopropylen-, Butylen-, Isobutylen-, sec.-Butylen- und tert.-Butylen-1,3-, -1,4-, -1,5- und -1,6-(2-butyl)phenylen, Methylen-, Ethylen-, Propylen-, Isopropylen-, Butylen-, Isobutylen-, sec.-Butylen- und tert.-Butylen-1,4-, -1,5- und -1,6-(2,3-dibutyl)phenylen, Methylen-, Ethylen-, Propylen-, Isopropylen-, Butylen-, Isobutylen-, sec.-Butylen- und tert.-Butylen-1,3-, -1,5- und -1,6-(2,4-dibutyl)phenylen, Methylen-, Ethylen-, Propylen-, Isopropylen-, Butylen-, Isobutylen-, sec.-Butylen- und tert.-Butylen-1,3-, -1,4- und -1,6-(2,5-dibutyl)phenylen, Methylen-, Ethylen-, Propylen-, Isopropylen-, Butylen-, Isobutylen-, sec.-Butylen- und tert.-Butylen-1,3- und -1,6-(2,4,5-tributyl)phenylen, Methylen-, Ethylen-, Propylen-, Isopropylen-, Butylen-, Isobutylen-, sec.-Butylen- und tert.-Butylen-1,3-(2,4,6-tributyl)phenylen, Methylen-, Ethylen-, Propylen-, Isopropylen-, Butylen-, Isobutylen-, sec.-Butylen- und tert.-Butylen-1,3-, -1,4-, -1,5- und -1,6-(2-isobutyl)phenylen, Methylen-, Ethylen-, Propylen-, Isopropylen-, Butylen-, Isobutylen-, sec.-Butylen- und tert.-Butylen-1,4-, -1,5- und -1,6-(2,3-diisobutyl)phenylen, Methylen-, Ethylen-, Propylen-, Isopropylen-, Butylen-, Isobutylen-, sec.-Butylen- und tert.-Butylen-1,3-, -1,5- und -1,6-(2,4-diisobutyl)phenylen, Methylen-, Ethylen-, Propylen-, Isopropylen-, Butylen-, Isobutylen-, sec.-Butylen- und tert.-Butylen-1,3-, -1,4- und -1,6-(2,5-diisobutyl)phenylen, Methylen-, Ethylen-, Propylen-, Isopropylen-, Butylen-, Isobutylen-, sec.-Butylen- und tert.-Butylen-1,3- und -1,6-(2,4,5-triisobutyl)phenylen, Methylen-, Ethylen-, Propylen-, Isopropylen-, Butylen-, Isobutylen-, sec.-Butylen- und tert.-Butylen-1,3-(2,4,6-triisobutyl)phenylen, Methylen-, Ethylen-, Propylen-, Isopropylen-, Butylen-, Isobutylen-, sec.-Butylen- und tert.-Butylen-1,3-, -1,4-, -1,5- und -1,6-(2-sec.-butyl)phenylen, Methylen-, Ethylen-, Propylen-, Isopropylen-, Butylen-, Isobutylen-, sec.-Butylen- und tert.-Butylen-1,4-, -1,5- und -1,6-(2,3-di-sec.-butyl)phenylen, Methylen-, Ethylen-, Propylen-, Isopropylen-, Butylen-, Isobutylen-, sec.-Butylen- und tert.-Butylen-1,3-, -1,5- und -1,6-(2,4-di-sec.-butyl)phenylen, Methylen-, Ethylen-, Propylen-, Isopropylen-, Butylen-, Isobutylen-, sec.-Butylen- und tert.-Butylen-1,3-, -1,4- und -1,6-(2,5-di-sec.-butyl)phenylen, Methylen-, Ethylen-, Propylen-, Isopropylen-, Butylen-, Isobutylen-, sec.-Butylen- und tert.-Butylen-1,3- und -1,6-(2,4,5-tri-sec.-butyl)phenylen, Methylen-, Ethylen-, Propylen-, Isopropylen-, Butylen-, Isobutylen-, sec.-Butylen- und tert.-Butylen-1,3-(2,4,6-tri-sec.-butyl)phenylen, Methylen-, Ethylen-, Propylen-, Isopropylen-, Butylen-, Isobutylen-, sec.-Butylen- und tert.-Butylen-1,3-, -1,4-, -1,5- und -1,6-(2-tert.-butyl)phenylen, Methylen-, Ethylen-, Propylen-, Isopropylen-, Butylen-, Isobutylen-, sec.-Butylen- und tert.-Butylen-1,4-, -1,5- und -1,6-(2,3-di-tert.-butyl)phenylen, Methylen-, Ethylen-, Propylen-, Isopropylen-, Butylen-, Isobutylen-, sec.-Butylen- und tert.-Butylen-1,3-, -1,5- und -1,6-(2,4-di-tert.-butyl)phenylen, Methylen-, Ethylen-, Propylen-, Isopropylen-, Butylen-, Isobutylen-, sec.-Butylen- und tert.-Butylen-1,3-, -1,4- und -1,6-(2,5-di-tert.-butyl)phenylen, Methylen-, Ethylen-, Propylen-, Isopropylen-, Butylen-, Isobutylen-, sec.-Butylen- und tert.-Butylen-1,3- und -1,6-(2,4,5-tri-tert.-butyl)phenylen, Methylen-, Ethylen-, Propylen-, Isopropylen-, Butylen-, Isobutylen-, sec.-Butylen- und tert.-Butylen-1,3-(2,4,6-tri-tert.-butyl)phenylen;
Methylen-, Ethylen-, Propylen-, Isopropylen-, Butylen-, Isobutylen-, sec.-Butylen- und tert.-Butylen-1,3-, -1,4-, -1,5- und -1,6-(2-methoxy)phenylen, Methylen-, Ethylen-, Propylen-, Isopropylen-, Butylen-, Isobutylen-, sec.-Butylen- und tert.-Butylen-1,4-, -1,5- und -1,6-(2,3-dimethoxy)phenylen, Methylen-, Ethylen-, Propylen-, Isopropylen-, Butylen-, Isobutylen-, sec.-Butylen- und tert.-Butylen-1,3-, -1,5- und -1,6-(2,4-dimethoxy)phenylen, Methylen-, Ethylen-, Propylen-, Isopropylen-, Butylen-, Isobutylen-, sec.-Butylen- und tert.-Butylen-1,3-, -1,4- und -1,6-(2,5-dimethoxy)phenylen, Methylen-, Ethylen-, Propylen-, Isopropylen-, Butylen-, Isobutylen-, sec.-Butylen- und tert.-Butylen-1,3- und -1,6-(2,4,5-trimethoxy)phenylen, Methylen-, Ethylen-, Propylen-, Isopropylen-, Butylen-, Isobutylen-, sec.-Butylen- und tert.-Butylen-1,3-(2,4,6-trimethoxy)phenylen, Methylen-, Ethylen-, Propylen-, Isopropylen-, Butylen-, Isobutylen-, sec.-Butylen- und tert.-Butylen-1,3-, -1,4-, -1,5- und -1,6-(2-ethoxy)phenylen, Methylen-, Ethylen-, Propylen-, Isopropylen-, Butylen-, Isobutylen-, sec.-Butylen- und tert.-Butylen-1,4-, -1,5- und -1,6-(2,3-diethoxy)phenylen, Methylen-, Ethylen-, Propylen-, Isopropylen-, Butylen-, Isobutylen-, sec.-Butylen- und tert.-Butylen-1,3-, -1,5- und -1,6-(2,4-diethoxy)phenylen, Methylen-, Ethylen-, Propylen-, Isopropylen-, Butylen-, Isobutylen-, sec.-Butylen- und tert.-Butylen-1,3-, -1,4- und -1,6-(2,5-diethoxy)phenylen, Methylen-, Ethylen-, Propylen-, Isopropylen-, Butylen-, Isobutylen-, sec.-Butylen- und tert.-Butylen-1,3- und -1,6-(2,4,5-triethoxy)phenylen, Methylen-, Ethylen-, Propylen-, Isopropylen-, Butylen-, Isobutylen-, sec.-Butylen- und tert.-Butylen-1,3-(2,4,6-triethoxy)phenylen, Methylen-, Ethylen-, Propylen-, Isopropylen-, Butylen-, Isobutylen-, sec.-Butylen- und tert.-Butylen-1,3-, -1,4-, -1,5- und -1,6-(2-propoxy)phenylen, Methylen-, Ethylen-, Propylen-, Isopropylen-, Butylen-, Isobutylen-, sec.-Butylen- und tert.-Butylen-1,4-, -1,5- und -1,6-(2,3-dipropoxy)phenylen, Methylen-, Ethylen-, Propylen-, Isopropylen-, Butylen-, Isobutylen-, sec.-Butylen- und tert.-Butylen-1,3-, -1,5- und -1,6-(2,4-dipropoxy)phenylen, Methylen-, Ethylen-, Propylen-, Isopropylen-, Butylen-, Isobutylen-, sec.-Butylen- und tert.-Butylen-1,3-, -1,4- und -1,6-(2,5-dipropoxy)phenylen, Methylen-, Ethylen-, Propylen-, Isopropylen-, Butylen-, Isobutylen-, sec.-Butylen- und tert.-Butylen-1,3- und -1,6-(2,4,5-tripropoxy)phenylen, Methylen-, Ethylen-, Propylen-, Isopropylen-, Butylen-, Isobutylen-, sec.-Butylen- und tert.-Butylen-1,3-(2,4,6-tripropoxy)phenylen, Methylen-, Ethylen-, Propylen-, Isopropylen-, Butylen-, Isobutylen-, sec.-Butylen- und tert.-Butylen-1,3-, -1,4-, -1,5- und -1,6-(2-isopropoxy)phenylen, Methylen-, Ethylen-, Propylen-, Isopropylen-, Butylen-, Isobutylen-, sec.-Butylen- und tert.-Butylen-1,4-, -1,5- und -1,6-(2,3-diisopropoxy)phenylen, Methylen-, Ethylen-, Propylen-, Isopropylen-, Butylen-, Isobutylen-, sec.-Butylen- und tert.-Butylen-1,3-, -1,5- und -1,6-(2,4-diisopropoxy)phenylen, Methylen-, Ethylen-, Propylen-, Isopropylen-, Butylen-, Isobutylen-, sec.-Butylen- und tert.-Butylen-1,3-, -1,4- und -1,6-(2,5-diisopropoxy)phenylen, Methylen-, Ethylen-, Propylen-, Isopropylen-, Butylen-, Isobutylen-, sec.-Butylen- und tert.-Butylen-1,3- und -1,6-(2,4,5-triisopropoxy)phenylen, Methylen-, Ethylen-, Propylen-, Isopropylen-, Butylen-, Isobutylen-, sec.-Butylen- und tert.-Butylen-1,3-(2,4,6-triisopropoxy)phenylen, Methylen-, Ethylen-, Propylen-, Isopropylen-, Butylen-, Isobutylen-, sec.-Butylen- und tert.-Butylen-1,3-, -1,4-, -1,5- und -1,6-(2-butoxy)phenylen, Methylen-, Ethylen-, Propylen-, Isopropylen-, Butylen-, Isobutylen-, sec.-Butylen- und tert.-Butylen-1,4-, -1,5- und -1,6-(2,3-dibutoxy)phenylen, Methylen-, Ethylen-, Propylen-, Isopropylen-, Butylen-, Isobutylen-, sec.-Butylen- und tert.-Butylen-1,3-, -1,5- und -1,6-(2,4-dibutoxy)phenylen, Methylen-, Ethylen-, Propylen-, Isopropylen-, Butylen-, Isobutylen-, sec.-Butylen- und tert.-Butylen-1,3-, -1,4- und -1,6-(2,5-dibutoxy)phenylen, Methylen-, Ethylen-, Propylen-, Isopropylen-, Butylen-, Isobutylen-, sec.-Butylen- und tert.-Butylen-1,3- und -1,6-(2,4,5-tributoxy)phenylen, Methylen-, Ethylen-, Propylen-, Isopropylen-, Butylen-, Isobutylen-, sec.-Butylen- und tert.-Butylen-1,3-(2,4,6-tributoxy)phenylen;
Methylen-, Ethylen-, Propylen-, Isopropylen-, Butylen-, Isobutylen-, sec.-Butylen- und tert.-Butylen-1,3-, -1,4-, -1,5- und -1,6-(2-cyano)phenylen, Methylen-, Ethylen-, Propylen-, Isopropylen-, Butylen-, Isobutylen-, sec.-Butylen- und tert.-Butylen-1,4-, -1,5- und -1,6-(2,3-dicyano)phenylen, Methylen-, Ethylen-, Propylen-, Isopropylen-, Butylen-, Isobutylen-, sec.-Butylen- und tert.-Butylen-1,3-, -1,5- und -1,6-(2,4-dicyano)phenylen, Methylen-, Ethylen-, Propylen-, Isopropylen-, Butylen-, Isobutylen-, sec.-Butylen- und tert.-Butylen-1,3-, -1,4- und -1,6-(2,5-dicyano)phenylen;
Methylen, Ethylen-, Propylen-, Isopropylen-, Butylen-, Isobutylen-, sec.-Butylen- und tert.-Butylen-1,2-, -1,3-, -1,4-, -1,5-, -1,6-, -1,7-, -1,8-, -2,6- und -2,7-naphthylen, Methylen, Ethylen-, Propylen-, Isopropylen-, Butylen-, Isobutylen-, sec.-Butylen- und tert.-Butylen-2,3-, -2,4-, -2,5-, -2,6-, -2,7-, -2,8-, -3,4-, -3,5-, -3,6-, -3,7-, -3,8-, -4,5-, -4,6-, -4,7-, -4,8-, -5,6-, -5,7-, -5,8-, -6,7-, -6,8- und -7,9-(1-methyl)naphthylen, Methylen, Ethylen-, Propylen-, Isopropylen-, Butylen-, Isobutylen-, sec.-Butylen- und tert.-Butylen-1,3-, -1,4-, -1,5-, -1,6-, -1,7-, -1,8-, -3,4-, -3,5-, -3,6-, -3,7-, -3,8-, -4,5-, -4,6-, -4,7-, -4,8-, -5,6-, -5,7-, -5,8-, -6,7-, -6,8- und -7,9-(2-methyl)naphthylen, Methylen, Ethylen-, Propylen-, Isopropylen-, Butylen-, Isobutylen-, sec.-Butylen- und tert.-Butylen-2,3-, -2,4-, -2,5-, -2,6-, -2,7-, -2,8-, -3,4-, -3,5-, -3,6-, -3,7-, -3,8-, -4,5-, -4,6-, -4,7-, -4,8-, -5,6-, -5,7-, -5,8-, -6,7-, -6,8- und -7,9-(1-ethyl)naphthylen, Methylen, Ethylen-, Propylen-, Isopropylen-, Butylen-, Isobutylen-, sec.-Butylen- und tert.-Butylen-1,3-, -1,4-, -1,5-, -1,6-, -1,7-, -1,8-, -3,4-, -3,5-, -3,6-, -3,7-, -3,8-, -4,5-, -4,6-, -4,7-, -4,8-, -5,6-, -5,7-, -5,8-, -6,7-, -6,8- und -7,9-(2-ethyl)naphthylen, Methylen, Ethylen-, Propylen-, Isopropylen-, Butylen-, Isobutylen-, sec.-Butylen- und tert.-Butylen-2,3-, -2,4-, -2,5-, -2,6-, -2,7-, -2,8-, -3,4-, -3,5-, -3,6-, -3,7-, -3,8-, -4,5-, -4,6-, -4,7-, -4,8-, -5,6-, -5,7-, -5,8-, -6,7-, -6,8- und -7,9-(1-propyl)naphthylen, Methylen, Ethylen-, Propylen-, Isopropylen-, Butylen-, Isobutylen-, sec.-Butylen- und tert.-Butylen-1,3-, -1,4-, -1,5-, -1,6-, -1,7-, -1,8-, -3,4-, -3,5-, -3,6-, -3,7-, -3,8-, -4,5-, -4,6-, -4,7-, -4,8-, -5,6-, -5,7-, -5,8-, -6,7-, -6,8- und -7,9-(2-propyl)naphthylen, Methylen, Ethylen-, Propylen-, Isopropylen-, Butylen-, Isobutylen-, sec.-Butylen- und tert.-Butylen-2,3-, -2,4-, -2,5-, -2,6-, -2,7-, -2,8-, -3,4-, -3,5-, -3,6-, -3,7-, -3,8-, -4,5-, -4,6-, -4,7-, -4,8-, -5,6-, -5,7-, -5,8-, -6,7-, -6,8- und -7,9-(1-isopropyl)naphthylen, Methylen, Ethylen-, Propylen-, Isopropylen-, Butylen-, Isobutylen-, sec.-Butylen- und tert.-Butylen-1,3-, -1,4-, -1,5-, -1,6-, -1,7-, -1,8-, -3,4-, -3,5-, -3,6-, -3,7-, -3,8-, -4,5-, -4,6-, -4,7-, -4,8-, -5,6-, -5,7-, -5,8-, -6,7-, -6,8- und -7,9-(2-isopropyl)naphthylen;
Methylen-, Ethylen-, Propylen-, Isopropylen-, Butylen-, Isobutylen-, sec.-Butylen- und tert.-Butylen-2,3-, -2,4-, -2,5-, -2,6-, -3,4- und -3,5-pyridylen, Methylen-, Ethylen-, Propylen-, Isopropylen-, Butylen-, Isobutylen-, sec.-Butylen- und tert.-Butylen-3,4-, -3,5-, -3,6-, -4,5-, -4,6- und -5,6-(2-methyl)pyridylen, Methylen-, Ethylen-, Propylen-, Isopropylen-, Butylen-, Isobutylen-, sec.-Butylen- und tert.-Butylen-3,4-, -3,5-, -3,6-, -4,5-, -4,6- und -5,6-(2-ethyl)pyridylen, Methylen-, Ethylen-, Propylen-, Isopropylen-, Butylen-, Isobutylen-, sec.-Butylen- und tert.-Butylen-3,4-, -3,5-, -3,6-, -4,5-, -4,6- und -5,6-(2-propyl)pyridylen, Methylen-, Ethylen-, Propylen-, Isopropylen-, Butylen-, Isobutylen-, sec.-Butylen- und tert.-Butylen-3,4-, -3,5-, -3,6-, -4,5-, -4,6- und -5,6-(2-isopropyl)pyridylen, Methylen-, Ethylen-, Propylen-, Isopropylen-, Butylen-, Isobutylen-, sec.-Butylen- und tert.-Butylen-2,4-, -2,5-, -2,6-, -4,5-, -4,6- und -5,6-(3-methyl)pyridylen, Methylen-, Ethylen-, Propylen-, Isopropylen-, Butylen-, Isobutylen-, sec.-Butylen- und tert.-Butylen-2,4-, -2,5-, -2,6-, -4,5-, -4,6- und -5,6-(3-ethyl)pyridylen, Methylen-, Ethylen-, Propylen-, Isopropylen-, Butylen-, Isobutylen-, sec.-Butylen- und tert.-Butylen-2,4-, -2,5-, -2,6-, -4,5-, -4,6- und -5,6-(3-propyl)pyridylen, Methylen-, Ethylen-, Propylen-, Isopropylen-, Butylen-, Isobutylen-, sec.-Butylen- und tert.-Butylen-2,4-, -2,5-, -2,6-, -4,5-, -4,6- und -5,6-(3-isopropyl)pyridylen, Methylen-, Ethylen-, Propylen-, Isopropylen-, Butylen-, Isobutylen-, sec.-Butylen- und tert.-Butylen-2,3-, -2,5-, -2,6-, -3,5-, -3,6- und -5,6-(4-methyl)pyridylen, Methylen-, Ethylen-, Propylen-, Isopropylen-, Butylen-, Isobutylen-, sec.-Butylen- und tert.-Butylen-2,3-, -2,5-, -2,6-, -3,5-, -3,6- und -5,6-(4-ethyl)pyridylen, Methylen-, Ethylen-, Propylen-, Isopropylen-, Butylen-, Isobutylen-, sec.-Butylen- und tert.-Butylen-2,3-, -2,5-, -2,6-, -3,5-, -3,6- und -5,6-(4-propyl)pyridylen, Methylen-, Ethylen-, Propylen-, Isopropylen-, Butylen-, Isobutylen-, sec.-Butylen- und tert.-Butylen-2,3-, -2,5-, -2,6-, -3,5-, -3,6- und -5,6-(4-isopropyl)pyridylen;
Methylen-, Ethylen-, Propylen-, Isopropylen-, Butylen-, Isobutylen-, sec.-Butylen- und tert.-Butylen-2,5- und 3,4-Pyrrylen, Methylen-, Ethylen-, Propylen-, Isopropylen-, Butylen-, Isobutylen-, sec.-Butylen- und tert.-Butylen-2,4- und 2,5-Pyrimidylen, Methylen-, Ethylen-, Propylen-, Isopropylen-, Butylen-, Isobutylen-, sec.-Butylen- und tert.-Butylen-2,3-, 2,5- und 2,6-Pyrazylen, Methylen-, Ethylen-, Propylen-, Isopropylen-, Butylen-, Isobutylen-, sec.-Butylen- und tert.-Butylen-3,5-Pyrazolylen, Methylen-, Ethylen-, Propylen-, Isopropylen-, Butylen-, Isobutylen-, sec.-Butylen- und tert.-Butylen-1,2-, 1,4-, 1,5-, 2,4- und 2,5-Imidazolylen, Methylen-, Ethylen-, Propylen-, Isopropylen-, Butylen-, Isobutylen-, sec.-Butylen- und tert.-Butylen-2,4- und 2,5-Thiazolylen, Methylen-, Ethylen-, Propylen-, Isopropylen-, Butylen-, Isobutylen-, sec.-Butylen- und tert.-Butylen-3,5- und 3,6-(1,2,4-Triazylen), Methylen-, Ethylen-, Propylen-, Isopropylen-, Butylen-, Isobutylen-, sec.-Butylen- und tert.-Butylen-2,4-(1,3,5-Triazylen), Methylen-, Ethylen-, Propylen-, Isopropylen-, Butylen-, Isobutylen-, sec.-Butylen- und tert.-Butylen-3,5-, 3,6-, 3,8- und 5,8-Chinaldylen, Methylen-, Ethylen-, Propylen-, Isopropylen-, Butylen-, Isobutylen-, sec.-Butylen- und tert.-Butylen-3,5-, 3,6-, 3,8- und 5,8-Chinolinylen, Methylen-, Ethylen-, Propylen-, Isopropylen-, Butylen-, Isobutylen-, sec.-Butylen- und tert.-Butylen-2,4- und 2,5-Benzimidazolylen und Methylen-, Ethylen-, Propylen-, Isopropylen-, Butylen-, Isobutylen-, sec.-Butylen- und tert.-Butylen-1,3-, 1,4-, 1,5-und 1,6-Isochinolylen.

Als Beispiele für die radikalisch polymerisierbare Gruppe P der allgemeinen Formel II sind insbesondere Vinyl, Allyl, Methallyl, Acrylamido, Methacrylamido, Acryloxy, Methacryloxy, 3-Vinylureido, 3-Allylureido, 3-Methallylureido, N-Vinylaminocarbonyloxy, N-Allylaminocarbonyloxy und N-Methallylaminocarbonyloxy zu nennen.

Die Herstellung der erfindungsgemäßen 9-cyanosubstituierten Perylen-3,4-dicarbonsäuremonoimide I kann vorteilhaft nach den drei erfindungsgemäßen Verfahren, ausgehend vom literaturbekannten Perylen-3,4-dicarbonsäureanhydrid oder den in den WO-A-96/22331 und WO-A-01/16109 beschriebenen 9-Bromperylen-3,4-dicarboximiden der Formel VI, erfolgen.

Zur Darstellung der erfindungsgemäßen nicht- bzw. monofunktionellen 9-Cyanoperylen-3,4-dicarbonsäuremonoimide Ia wird zunächst in einem Schritt a) Perylen-3,4-dicarbonsäureanhydrid mit elementarem Brom selektiv in der 9-Position bromiert, in einem Schritt b) wird das in Schritt a) erhaltene 9-Bromperylen-3,4-dicarbonsäureanhydrid IIIa mit Kupfer(I)cyanid im Überschuß umgesetzt, und in einem abschließenden Schritt c) wird das in Schritt b) erhaltene 9-Cyanoperylen-3,4-dicarbonsäureanhydrid IIIa mit einem primären Amin IV zu den gewünschten 9-Cyanoperylen-3,4-dicarbonsäuremonoimiden Ia umgesetzt.

Schritt a) des erfindungsgemäßen Herstellungsverfahrens, die Umsetzung von Perylen-3,4-dicarbonsäureanhydrid mit elementarem Brom, wird in Gegenwart von konzentrierter Schwefelsäure oder einer aliphatischen Monocarbonsäure als Lösungsmittel vorgenommen.

Als Beispiele für geeignete aliphatische Monocarbonsäuren seien insbesondere 2 bis 6 C-Atome aufweisende Carbonsäuren, also im einzelnen Essigsäure, Propionsäure, Buttersäure, Pentancarbonsäure und Hexancarbonsäure sowie Mischungen derselben genannt.

Üblicherweise kommen 10 bis 50 g, vorzugsweise 15 bis 30 g, Lösungsmittel je g zu bromierendes Perylen-3,4-dicarbonsäureanhydrid zum Einsatz.

In der Regel ist die Anwesenheit eines Halogenierungskatalysators nicht erforderlich. Will man jedoch die Bromierungsreaktion beschleunigen (etwa um den Faktor 2 bis 5), so empfiehlt es sich, elementares Iod, bevorzugt in einer Menge von 1 bis 5 mol-%, bezogen auf Perylen-3,4-dicarbonsäureanhydrid, zuzusetzen.

Im allgemeinen liegt das Molverhältnis von Brom zu Perylen-3,4-dicarbonsäureanhydrid bei etwa 0,8:1 bis 2:1, vorzugsweise bei 1,0:1 bis 1,5:1.

Die Reaktionstemperatur beträgt in der Regel 0 bis 70°C, insbesondere 0 bis 10°C bei Verwendung von konz. Schwefelsäure und 20 bis 60°C bei Verwendung aliphatischer Carbonsäuren als Lösungsmittel.

In Abhängigkeit von der Reaktionstemperatur und der An- oder Abwesenheit von Iod ist die Bromierung üblicherweise in 2 bis 12 h beendet.

Verfahrenstechnisch geht man in Schritt a) zweckmäßigerweise wie folgt vor:
Man legt Lösungsmittel und Perylen-3,4-dicarbonsäureanhydrid vor, setzt gegebenenfalls den Katalysator und anschließend in 5 bis 10 min die gewünschte Brommenge zu, bringt die Mischung unter Rühren auf die gewünschte Reaktionstemperatur, rührt 2 bis 12 h bei Reaktionstemperatur nach und entfernt überschüssiges Brom mit einem kräftigen Stickstoffstrom. Bei Verwendung von Schwefelsäure trägt man die Reaktionsmischung in das zehnfache Volumen an Eiswasser, ein, filtriert das ausgefallene Produkt ab, wäscht mit Wasser bis zum neutralen Ablauf und trocknet unter Vakuum bei etwa 120°C. Bei Verwendung von aliphatischen Monocarbonsäuren als Solvens kühlt man die Reaktionsmischung auf Raumtemperatur ab, verdünnt gegebenenfalls mit dem doppelten Volumen eines aliphatischen Alkohols wie Methanol, filtriert den ausgefallenen Niederschlag ab, rührt ihn nacheinander in Methanol und 2 gew.-%iger Natriumthiosulfatlösung auf, filtriert erneut ab, wäscht mit heißem Wasser bis zum neutralen und salzfreien Ablauf und trocknet bei 120°C im Vakuum.

Die Reinheit des so hergestellten, ebenfalls erfindungsgemäßen 9-Bromperylendicarbonsäureanhydrids IIIa liegt in der Regel bei > 97% und reicht somit im allgemeinen für die Weiterverarbeitung aus. Gewünschtenfalls kann eine Aufreinigung durch Säulenfiltration an Kieselgel mit Methylenchlorid als Eluens oder durch Extraktion mit einem Lösungsmittel wie Methanol durchgeführt werden.

In Schritt b) des erfindungsgemäßen Verfahrens erfolgt die Umsetzung des in Schritt a) erhaltenen 9-Bromperylen-3,4-dicarbonsäureanhydrids IIIa in einem hochsiedenden inerten Verdünnungsmittel, gegebenenfalls unter Zusatz einer stickstoffbasischen Verbindung oder eines Stickstoffheterocyclus als Katalysator, mit Kupfer(I)cyanid im Überschuß.

Als hochsiedende, inerte Verdünnungsmittel eignen sich hierfür insbesondere (Bi)Cycloaliphaten, wie Cyclohexan und seine alkylierten Derivate sowie Decahydronaphthalin, cylische Sulfoxide, wie Sulfolan, und polare Lösungsmittel, vor allem Stickstoffheterocyclen, wie Pyridin, Pyrimidin, Chinolin, Isochinolin, Chinaldin und N-Methylpyrrolidon, sowie Carbonsäureamide, wie N,N-Dimethylformamid und N,N-Dimethylacetamid, wobei Sulfolan, N-Methylpyrrolidon und N,N-Dimethylacetamid bevorzugt sind. Die letzten beiden Verbindungsklassen können auch gleichzeitig als Katalysator dienen.

Üblicherweise kommen 10 bis 50 g, vorzugsweise 10 bis 25 g, Verdünnungsmittel je g 9-Bromperylen-3,4-dicarbonsäureanhydrid IIIa zum Einsatz.

Bei Verwendung von (Bi)Cycloaliphaten als hochsiedende, inerte Verdünnungsmittel kann die Substitutionsreaktion durch Zusatz von 5 bis 10 Vol.-% einer stickstoffbasischen Verbindung oder eines Stickstoffheterocyclus als Katalysator deutlich beschleunigt werden; in allen anderen Fällen erübrigt sich ein solcher Zusatz.

Im allgemeinen werden 1 bis 3, bevorzugt 1,5 bis 2,5, mol Kupfer(I)cyanid je mol zu substituierendes Bromatom eingesetzt.

Die Reaktionstemperatur beträgt in der Regel 150 bis 350°C, insbesondere 180 bis 300°C.

In Abhängigkeit von der Reaktionstemperatur ist die Substitutionsreaktion üblicherweise in 0,5 bis 6 h beendet.

Verfahrenstechnisch geht man in Schritt b) zweckmäßigerweise wie folgt vor:
Eine Mischung aus 9-Bromperylen-3,4-dicarbonsäureanhydrid IIIa, Kupfer(I)cyanid, Verdünnungsmittel und gegebenenfalls stickstoffbasischem Katalysator wird unter Rühren in einer Stickstoffatmosphäre 0,5 bis 6 h auf die gewünschte Reaktionstemperatur erhitzt. Nach dem Abkühlen der Reaktionsmischung wird mit dem doppelten bis dreifachen Volumen eines aliphatischen Alkohols wie Methanol oder aber einer Alkohol/Wasser-Mischung verdünnt, der ausgefallene Niederschlag abfiltriert, zur Zersetzung überschüssigen Kupfer(I)cyanids in einer salzsauren Eisen(III)chloridlösung gerührt, erneut filtriert, der Rückstand mit heißem Wasser bis zum neutralen, fast farblosen Ablauf gewaschen und bei 120°C im Vakuum getrocknet.

Die Reinheit des so hergestellten, ebenfalls erfindungsgemäßen 9-Cyanoperylendicarbonsäureanhydrids IIIb liegt üblicherweise bei > 95% und reicht somit im allgemeinen für die Weiterverarbeitung aus. Gewünschtenfalls kann analog zum 9-Bromperylendicarbonsäureanhydrid IIIa eine Aufreinigung durch Säulenfiltration an Kieselgel mit Methylenchlorid als Eluens oder durch Extraktion mit einem Lösungsmittel wie Methanol durchgeführt werden.

Schritt c) des erfindungsgemäßen Herstellungsverfahrens, die Umsetzung des in Schritt b) erhaltenen 9-Cyanoperylen-3,4-dicarbonsäureanhydrids IIIb mit dem primären Amin IV zu dem gewünschten 9-Cyanoperylen-3,4-dicarbonsäuremonoimiden Ia, erfolgt in Wasser oder einem geeigneten inerten organischen Lösungsmittel, gegebenenfalls unter Zusatz eines Imidierungskatalysators.

Abhängig vom Löslichkeitsverhalten, der Polarität und der Reaktivität des eingesetzten primären Amins IV finden entweder Wasser oder aber inerte organische Lösungsmittel Verwendung. Kurzkettige, aliphatische und cycloaliphatische Amine mit hoher Reaktivität können vorteilhaft in Wasser als Lösungsmittel umgesetzt werden, während langkettige aliphatische und aromatische Amine mittlerer und niedriger Reaktivität und eingeschränkter Wasserlöslichkeit den Einsatz mehr oder weniger polarer, inerter organischer Lösungsmittel erforderlich machen.

Als inerte organische Lösungsmittel eignen sich Aromaten, wie Toluol und Xylol (alle Isomere), (Bi)Cycloaliphaten, wie alkylsubstituiertes Cyclohexan und Decahydronaphthalin, sowie insbesondere polare stickstoffbasische Verbindungen, vor allem Stickstoffheterocyclen, wie Pyridin, Pyrimidin, Chinolin, Isochinolin, Chinaldin und N-Methylpyrrolidon, sowie Carbonsäureamide, wie N,N-Dimethylformamid und N,N-Dimethylacetamid, wobei N-Methylpyrrolidon bevorzugt ist.

Die Menge an Lösungsmittel ist an sich nicht kritisch, üblicherweise kommen 10 bis 100 g, vorzugsweise 20 bis 50 g, Lösungsmittel je g IIIb zum Einsatz.

Für die Umsetzung reaktionträger, insbesondere sterisch gehinderter primärer Amine ist der Zusatz eines Imidierungskatalysators erforderlich.

Als Imidierungskatalysatoren eignen sich organische und anorganische Salze von Übergangsmetallen wie Zink, Eisen und Kupfer, und von Magnesium, z.B. Zinkacetat, Zinkpropionat, Zinkoxid, Eisen(II)acetat, Eisen(III)chlorid, Eisen(II)sulfat, Kupfer(II)acetat, Kupfer(I)oxid, Kupfer(II)oxid und Magnesiumacetat. Selbstverständlich kann man auch Mischungen der genannten Katalysatoren verwenden.

In der Regel werden 5 bis 40 Gew.-%, bevorzugt 5 bis 15 Gew.-%, Katalysator, bezogen auf IIIb, eingesetzt.

Als primäre Amine IV können bei dem erfindungsgemäßen Herstellungsverfahren alle unter den Reaktionsbedingungen stabilen Amine eingesetzt werden, die mit Perylen-3,4-dicarbonsäureanhydriden Imide bilden.

Üblicherweise liegt das Molverhältnis von Amin IV zu IIIb, abhängig von der Reaktivität des Amins, bei 0,8:1 bis 5:1.

Die Reaktionstemperatur beträgt in Schritt c) im allgemeinen 40 bis 250°C. Bei der Umsetzung nichtfunktioneller, kurzkettig aliphatischer oder cycloaliphatischer Amine hoher Reaktivität sind Temperaturen von 40 bis 100°C, bei der Umsetzung nicht- und monofunktioneller aliphatischer oder alkylaromatischer Amine mittlerer bis hoher Reaktivität Temperaturen von 120 bis 200°C und bei der Umsetzung reaktionsträger, nicht- und monofunktioneller aromatischer Amine Temperaturen von 180 bis 250°C bevorzugt.

Es empfiehlt sich, unter Verwendung einer Schutzgasatmosphäre (z.B. Stickstoff) zu arbeiten.

Man kann Schritt c) des erfindungsgemäßen Verfahrens bei Normaldruck oder bei einem Überdruck von üblicherweise bis zu 10 bar durchführen. Die Arbeitsweise unter Druck ist vor allem beim Einsatz flüchtiger Amine (Siedepunkt ≤ etwa 180°C) zweckmäßig.

Üblicherweise ist die Umsetzung in 1 bis 10 h, vor allem in 1 bis 5 h, beendet.

Verfahrenstechnisch geht man in Schritt c) zweckmäßigerweise wie folgt vor:
Man legt das Amin IV, Lösungsmittel und gegebenenfalls Katalysator vor, fügt portionsweise das 9-Cyanoperylen-3,4-dicarbonsäureanhydrid IIIb bei Raumtemperatur zu, spült die Apparatur etwa 15 min mit Stickstoff, erhitzt das Gemisch unter Rühren auf die Reaktionstemperatur und hält es etwa 1 bis 5 h bei dieser Temperatur. Nach dem Abkühlen auf Raumtemperatur wird gegebenenfalls die Hälfte des Lösungsmittels im Vakuum abgezogen, das ausgefallene Reaktionsprodukt abfiltriert, mit einem aliphatischen Alkohol wie Methanol gewaschen und getrocknet.

Soll die Umsetzung unter Druck vorgenommen werden, so verwendet man eine Druckapparatur als Reaktionsgefäß, auf die man nach dem Einfüllen der Komponenten einen Stickstoffdruck von etwa 1 bis 2 bar gibt, erhitzt anschließend die gewünschte Zeit unter dem sich entwickelnden Eigendruck auf die Reaktionstemperatur und entspannt nach dem Abkühlen.

Die Reinheit der so hergestellten, erfindungsgemäßen 9-Cyanoperylen-3,4-dicarbonsäureimide Ia liegt in der Regel bei > 98% und reicht somit für eine Weiterverarbeitung aus. Gewünschtenfalls kann eine weitere Aufreinigung durch Säulenfiltration an Kieselgel mit einem halogenierten Kohlenwasserstoff, wie Methylenchlorid, Chloroform oder Tetrachlorethan, als Eluens oder über eine Klärfiltration einer heißen Lösung des Imids Ia in N-Methylpyrrolidon (NMP) und anschließende Ausfällung des Wertproduktes durch Zusatz eines wäßrigen aliphatischen Alkohols, wie Methanol oder Ethanol, durchgeführt werden.

Zur Darstellung der ebenfalls erfindungsgemäßen, eine radikalisch polymerisierbare Gruppe P enthaltenden 9-Cyanoperylen-3,4-dicarbonsäuremonoimide Ib werden in einem weiteren Schritt analog zu der in WO-A-99/40123 beschriebenen Vorgehensweise monofunktionelle 9-Cyanoperylendicarbonsäuremonoimide der Formel Ia, in der Y' Amino oder Hydroxy bedeutet, in einem inerten aprotischen Verdünnungsmittel unter Zusatz einer Stickstoffbase mit den Carbonsäurechloriden V umgesetzt.

Als inerte aprotische Verdünnungsmittel eignen sich aliphatische und cycloaliphatische Ether, wie Diethyl-, Dipropyl- und Dibutylether, Dimethoxy- und Diethoxyethan sowie Tetrahydrofuran und Dioxan, Aromaten, wie Toluol oder Xylol (alle Isomere), (Bi)Cycloaliphaten, wie alkylsubstituiertes Cyclohexan und Decahydronaphthalin, sowie insbesondere polare stickstoffbasische Verbindungen, vor allem Stickstoffheterocyclen, wie Chinolin, Isochinolin, Chinaldin und N-Methylpyrrolidon, sowie Carbonsäureamide, wie N,N-Dimethylformamid und N,N-Dimethylacetamid, wobei Dioxan und N,N-Dimethylformamid bevorzugt sind.

Vorzugsweise führt man die Umsetzung der Säurechloride V in Gegenwart zumindest äquimolarer Mengen einer Stickstoffbase durch, die auch als Cosolvens dienen kann. Bevorzugte Stickstoffbasen sind Trialkylamine, wie Triethylamin, Tripropylamin und Tributylamin, sterisch gehinderte Bicyclen, wie Diazabicyclooctan, Diazabicyclononen (DBN) und Diazabicycloundecen (DBU), und Stickstoffheterocyclen, wie Pyridin und Pyrimidin, wobei DBU und Pyridin bevorzugt sind.

Üblicherweise liegt das Molverhältnis von Carbonsäurechlorid V zu 9-Cyanoperylendicarbonsäuremonoimid Ia, abhängig von der Reaktivität des 9-Cyanoperylen-3,4-dicarbonsäureimids Ia, bei 1:1 bis 5:1.

Die Reaktionstemperatur liegt üblicherweise bei 20 bis 150°C, abhängig von der Reaktivität des 9-Cyanoperylen-3,4-dicarbonsäureimids Ia und der Polymerisationsneigung des gewünschten Endproduktes Ib. So liegt sie z.B. bei der Umsetzung von hydroxyfunktionellen 9-Cyanoperylendicarbonsäuremonoimiden Ia mit Acrylsäurechloriden bevorzugt bei 20 bis 70°C, während bei der Umsetzung mit Methacrylchloriden Temperaturen von 40 bis 90°C bevorzugt sind.

Üblicherweise ist die Umsetzung in 1 bis 5 h, vor allem in 1 bis 2 h, beendet.

Verfahrenstechnisch geht man zweckmäßigerweise wie folgt vor:
Man legt eine Lösung bzw. Dispersion des monofunktionellen 9-Cyanoperylendicarbonsäuremonoimids Ia im Verdünnungsmittel oder gegebenenfalls der Stickstoffbase vor, erhitzt auf die gewünschte Reaktionstemperatur, tropft langsam eine Lösung des Säurechlorids V im Verdünnungsmittel zu und rührt 1 bis 2 h bei dieser Temperatur. Nach der destillativen Entfernung von Verdünnungsmittel und überschüssiger Stickstoffbase unter Vakuum wird der Rückstand in Chloroform gelöst und einer Säulenfiltration über Kieselgel als stationärer Phase unterworfen. Abschließend wird das Lösungsmittel im Vakuum abgezogen.

Die Reinheit der so hergestellten, erfindungsgemäßen 9-Cyanoperylendicarbonsäureimide Ib liegt in der Regel bei > 98% und reicht somit für eine Weiterverarbeitung aus.

Zur Darstellung der ebenfalls erfindungsgemäßen, nichtfunktionellen 9-Cyanoperylendicarbonsäuremonoimide Ic wird ein gemäß der in den WO-A-96/22331 und WO-A-01/16109 beschriebenen Verfahren dargestelltes 9-Bromperylen-3,4-dicarbonsäuremonoimid VI durch Reaktion mit Kupfer(I)cyanid in Substanz oder in einem hochsiedenden inerten Verdünnungsmittel, gegebenenfalls unter Zusatz einer stickstoffbasischen Verbindung oder eines Stickstoffheterocyclus als Katalysator, in das gewünschte 9-Cyanoperylen-3,4-dicarbonsäuremonoimid Ic überführt.

Als hochsiedende, inerte Verdünnungsmittel eignen sich hierfür insbesondere (Bi)Cycloaliphaten, wie Cyclohexan und seine alkylierten Derivate sowie Decahydronaphthalin, cylische Sulfoxide, wie Sulfolan, und polare Lösungsmittel, vor allem Stickstoffheterocyclen, wie Pyridin, Pyrimidin, Chinolin, Isochinolin, Chinaldin und N-Methylpyrrolidon, sowie Carbonsäureamide, wie N,N-Dimethylformamid und N,N-Dimethylacetamid, wobei Sulfolan, N-Methylpyrrolidon und N,N-Dimethylacetamid bevorzugt sind. Die letzten beiden Verbindungsklassen können auch gleichzeitig als Katalysator dienen.

Üblicherweise kommen 5 bis 30 g, vorzugsweise 5 bis 15 g, Verdünnungsmittel je g 9-Bromperylen-3,4-dicarbonsäuremonoimid VI zum Einsatz.

Bei Umsetzung in Substanz ist der Zusatz von 20 bis 30 Gew.-% einer stickstoffbasischen Verbindung oder eines Stickstoffheterocyclus als Katalysator zur Durchführung der Reaktion unbedingt erforderlich. Auch bei Verwendung von (Bi)Cycloaliphaten als hochsiedende, inerte Verdünnungsmittel kann die Substitutionsreaktion durch Zusatz von 5 bis 10 Vol.-% einer stickstoffbasischen Verbindung oder eines Stickstoffheterocyclus als Katalysator deutlich beschleunigt werden; in allen anderen Fällen erübrigt sich ein solcher Zusatz.

Im allgemeinen werden 1 bis 3, bevorzugt 1,2 bis 2,0, mol Kupfer(I)cyanid je mol zu substituierendes Bromatom eingesetzt.

Die Reaktionstemperatur beträgt in der Regel 150 bis 350°C, insbesondere 180 bis 300°C.

In Abhängigkeit von der Reaktionstemperatur ist die Substitutionsreaktion üblicherweise in 0,5 bis 4 h beendet.

Verfahrenstechnisch geht man zweckmäßigerweise wie folgt vor:
Eine Mischung aus 9-Bromperylen-3,4-dicarbonsäuremonoimid VI, Kupfer(I)cyanid, Verdünnungsmittel und gegebenenfalls stickstoffbasischem Katalysator wird unter Rühren in einer Stickstoffatmosphäre 0,5 bis 4 h auf die gewünschte Reaktionstemperatur erhitzt. Nach dem Abkühlen der Reaktionsmischung wird mit dem gleichen bis doppelten Volumen eines aliphatischen Alkohols wie Methanol oder aber einer Alkohol/Wasser-Mischung verdünnt, der ausgefallene Niederschlag abfiltriert, zur Zersetzung überschüssigen Kupfer(I)cyanids in einer salzsauren Eisen(III)chloridlösung gerührt, erneut filtriert, der Rückstand mit heißem Wasser bis zum neutralen, fast farblosen Ablauf gewaschen und bei 120°C im Vakuum getrocknet.

Das so hergestellte Rohprodukt wird zur Reinigung in 70 bis 100°C warmem N-Methylpyrrolidon (NMP) gelöst, mit Aktivkohle versetzt, 30 min nachgerührt und heiß filtriert. Nach dem Abkühlen auf 60°C wird das Produkt durch Zusatz von 60 bis 65 vol.-%igem Ethanol ausgefällt, 3 h bei 60°C nachgerührt, langsam auf Raumtemperatur abgekühlt, abfiltriert, mit Ethanol NMP-frei gewaschen und getrocknet.

In der Regel haben die erfindungsgemäß erhaltenen 9-Cyanoperylendicarbonsäuremonoimide bereits einen so hohen Wertgehalt (> 98%), daß auf eine weitere Reinigung verzichtet werden kann. Analysenreine Produkte können durch Umkristallisation aus aromatischen Lösungsmitteln, wie Toluol und Xylol, oder halogenierten Kohlenwasserstoffen, wie Methylenchlorid und Chloroform, oder durch Filtration einer Lösung der Produkte in diesen Lösungsmitteln über Kieselgel und anschließendes Einengen hergestellt werden.

Mit Hilfe der erfindungsgemäßen Herstellungsverfahren können die 9-cyanosubstituierten Perylen-3,4-dicarbonsäuremonoimide I auf verfahrenstechnisch einfache, wirtschaftliche Weise in hohen Reinheiten (im allgemeinen > 98%) und guten Ausbeuten erhalten werden.

Die erfindungsgemäßen 9-cyanosubstituierten Perylen-3,4-dicarbonsäuremonoimide der Formeln Ia und Ic eignen sich vorteilhaft als Fluoreszenzfarbmittel, zur Einfärbung von hochmolekularen organischen und anorganischen Materialien, insbesondere von Kunststoffen, Lacken, Druckfarben, anorganisch-organischen Compositen und oxidischen Schichtsystemen, als Dispergierhilfsmittel, Pigmentadditive für organische Pigmente und Zwischenprodukte für die Herstellung von Fluoreszenzfarbmitteln und Pigmentadditiven, zur Herstellung von im gelben Bereich des elektromagnetischen Spektrums absorbierenden und/oder emittierenden wäßrigen Polymerisatdispersionen und Ink-Jet-Tinten, als Photoleiter in der Elektrophotographie, als farbgebende oder farbkorrigierende Komponente in emissiven und transflektiven Farbfiltern und in retroreflektiven Vorrichtungen, als Emitter in Elektro- und Chemilumineszenzanwendungen, als Aktivkomponenten in der Fluoreszenzkonversion, in Fluoreszenzsolarkollektoren, in Biolumineszenzarrays sowie in der Photovoltaik und als Laserfarbstoffe.

Die ebenfalls erfindungsgemäßen, polymerisierbaren 9-cyanosubstituierten Perylen-3,4-dicarbonsäuremonoimide der Formel Ib eignen sich insbesondere als Fluoreszenzfarbmittel, zur Reaktiveinfärbung von hochmolekularen organischen und anorganischen Materialien, insbesondere von Lacken, Druckfarben und anorganisch-organischen Compositen, zur Herstellung von ausblutechten, im gelben Bereich des elektromagnetischen Spektrums absorbierenden und/oder emittierenden wäßrigen Polymerisatdispersionen, als farbgebende oder farbkorrigierende Komponente in emissiven und transflektiven Farbfiltern und in retroreflektiven Vorrichtungen sowie als migrationsstabilisierter Emitter in Elektro- und Chemilumineszenzanwendungen.

### Beispiele

### a) Herstellung von 9-Bromperylen-3,4-dicarbonsäureanhydrid IIIa

### Beispiel 1

10 g (30 mmol) Perylen-3,4-dicarbonsäureanhydrid wurden unter Rühren bei 0-10°C in 200 g Schwefelsäure gelöst, zunächst mit 0,2 g Iod als Katalysator und anschließend in 10 min mit 5 g (30 mmol) Brom versetzt. Nach 4stündigem Rühren bei dieser Temperatur und Ausblasen von überschüssigem Brom mit Stickstoff wurde das Reaktionsgemisch auf 1000 ml Eiswasser gefällt. Das ausgefallene Produkt wurde abfiltriert, mit Wasser bis zum neutralen Ablauf gewaschen und bei 120°C im Vakuum getrocknet.

Es wurden 12,0 g 9-Bromperylen-3,4-dicarbonsäureanhydrid IIIa in Form eines rotbraunen, amorphen Feststoffs mit einem Schmelzpunkt von > 300°C erhalten, was einer Ausbeute von 96% entspricht.

### Analytische Daten:

Elementaranalyse (Gew.-% ber./gef.):
C: 65,85/65,7; H: 2,25/2,25; 0: 11,95/12,2; Br: 19,95/19,85;
Masse (FD, 8kV): m/z = 400,4 [M⁺, 100%];
IR (KBr): ν = 1750 (s, C=O), 1725 (s, C=O) cm⁻¹;
UV/VIS (CHCl₃) : λₘₐₓ (ε) = 487 (9750), 511 (31330) nm.

### Beispiel 2

Eine Mischung aus 10 g (30 mmol) Perylen-3,4-dicarbonsäureanhydrid, 0,2 g Iod als Katalysator, 7,5 g (45 mmol) Brom (zugetropft innerhalb von 10 min) und 250 ml Eisessig wurde unter Rühren 8 h auf 50°C erhitzt. Nach Abkühlen der Reaktionslösung auf Raumtemperatur und Ausblasen von überschüssigem Brom mit Stickstoff wurde der Reaktionsansatz mit 500 ml Methanol verdünnt. Der ausgefallene Niederschlag wurde abfiltriert, in 150 ml Methanol aufgeschlämmt, 1 h bei Raumtemperatur gerührt, abfiltriert, 2 h in 500 ml 2 gew.-%iger Natriumthiosulfatlösung gerührt, abfiltriert, mit 70°C warmem Wasser bis zum neutralen, fast farblosen Ablauf gewaschen und bei 120°C im Vakuum getrocknet.

Es wurden 11,3 g 9-Bromperylen-3,4-dicarbonsäureanhydrid IIIa in Form eines rotbraunen, amorphen Feststoffs mit einem Schmelzpunkt von > 300°C erhalten, was einer Ausbeute von 94% entspricht.

### b) Herstellung von 9-Cyanoperylen-3,4-dicarbonsäureanhydrid IIIb

### Beispiele 3 bis 5

Eine Mischung aus 10 g (25 mmol) 9-Bromperylen-3,4-dicarbonsäureanhydrid IIIa, 4,5 g (50 mmol) Kupfer(I)cyanid und 120 ml des Verdünnungsmittels V wurde unter Rühren in einer Stickstoffatmosphäre t h auf T°C erhitzt. Nach dem Abkühlen der Reaktionsmischung auf 60°C (Beispiel 3) bzw. Raumtemperatur (Beispiele 4 und 5) wurde mit 300 ml Methanol (Beispiel 3) bzw. einer Mischung aus Methanol und Wasser im Volumenverhältnis 1:3 (Beispiele 4 und 5) verdünnt. Der ausgefallene Niederschlag wurde abfiltriert, in eine Mischung aus 25 g Eisen(III)chlorid-Hexahydrat, 100 ml Wasser und 20 ml konz. Salzsäure eingetragen, die entstandene Suspension zunächst 2 h bei Raumtemperatur und anschließend 1 h bei 70°C gerührt, erneut filtriert, der Rückstand mit 70°C warmem Wasser bis zum neutralen, fast farblosen Ablauf gewaschen und bei 120°C im Vakuum getrocknet.

Weitere Einzelheiten zu diesen Versuchen sowie deren Ergebnisse sind in Tabelle 1 zusammengestellt.

**Tabelle 1**

| | | | | | | | |
|---|---|---|---|---|---|---|---|
| Bsp | Verdünnungsmittel V | t | T | Ausbeute/Reinheit | | | Aussehen |

| | [h] | [°C] | | [g (%)] / [%] | | | |
|---|---|---|---|---|---|---|---|
| 3 | Sulfolan | 1 | 280 | 7,5 | (86)/> | 97 | braun, mikrokristallin |
| 4 | N-Methylpyrrolidon | 3 | 200 | 7,65 | (88)/> | 95 | braun, amorph |
| 5 | N,N-Dimethylacetamid | 6 | 165 | 7,4 | (85)/> | 95 | braun, amorph |

### Analytische Daten:

Schmelzpunkt : > 300°C;
Elementaranalyse (Gew.-% ber./gef.):
C: 79,55/79,2; H: 2,6/2,6; N: 4,05/4,1; 0: 13,8/14,1;
Masse (FD, 8kV): m/z = 347,2 [M⁺, 100%];
IR (KBr): ν = 2200 (m, C≡N), 1760 (s, C=O), 1725 (s, C=O) cm⁻¹;
UV/VIS (CHCl₃) : λₘₐₓ (ε) = 473 (14870), 496 (39750) nm.

### c) Herstellung der 9-Cyanoperylen-3,4-dicarbonsäureimide Ia

### Beispiele 6 bis 16

x g (X mmol) Amin IV und y g (Y mmol) Katalysator K wurden unter Rühren in v ml Lösungsmittel L vorgelegt und bei Raumtemperatur portionsweise mit 6,95 g (20 mmol) 9-Cyanoperylen-3,4-dicarbonsäureanhydrid IIIb versetzt. Nach 15minütigem Spülen der Apparatur mit Stickstoff wurde das Gemisch unter Rühren t h auf die Reaktionstemperatur T°C erhitzt. Nach dem Abkühlen auf Raumtemperatur wurde das ausgefallene Reaktionsprodukt entweder direkt (Beispiele 6,12, 14-16 oder aber nach Abziehen der Hälfte des Lösungsmittels im Vakuum (Beispiele 7-11, 13) abfiltriert, mit viel Methanol gewaschen und bei 100°C im Vakuum getrocknet.

Weitere Einzelheiten zu diesen Versuchen sowie deren Ergebnisse sind in Tabelle 2 zusammengestellt.

Dabei bedeuten:
- ZnAc:: Zinkacetatdihydrat
- DMAc:: N,N-Dimethylacetamid
- NMP:: N-Methylpyrrolidon

**Tabelle 2**

| Bsp | Amin IV | x [g] | X [mmol] | Kat. K | y [g] | Y [mmol] | L | v [ml] | t [h] | Ausbeute [g /%] | Aussehen | Smp. [°C] |
|---|---|---|---|---|---|---|---|---|---|---|---|---|
| 6 | Methylamin | 0,9 | 30 | - | - | - | H₂O | 300 | 5 | 6,2 / 86 | rotbraun, kristallin | >300 |
| 7 | n-Dodecylamin | 4,1 | 22 | - | - | - | DMAc | 100 | 1,5 | 9,1 / 88 | rotbraun, amorph | 219 - 221 |
| 8 | 5-Nonylamin | 3,6 | 25 | - | - | - | NMP | 150 | 2 | 7,5 /79 | rotorange, amorph | 203 - 204 |
| 9 | 2,6-Diisopropylanilin | 7,1 | 40 | ZnAc | 1,0 | 4,5 | NMP | 200 | 3 | 8,6 / 85 | orangerot, mikrokristallin | >300 |
| 10 | 2,5-Di-tert.-butylanilin | 8,2 | 40 | ZnAc | 1,0 | 4,5 | NMP | 200 | 3 | 9,3 / 87 | orange, mikrokristallin | >300 |
| 11 | p-Methoxybenzylamin | 3,45 | 25 | - | - | - | NMP | 200 | 2 | 7,65 / 82 | braunorange, amorph | >300 |
| 12 | 5-Amino-1-pentanol | 2,3 | 22 | - | - | - | NMP | 200 | 1,5 | 6,85 / 79 | braunrot, mikrokristallin | 282 - 284 |
| 13 | Hexamethylendiamin | 11,6 | 100 | - | - | - | NMP | 200 | 3 | 6,7 / 75 | braun, amorph | 273 - 275 |
| 14 | 2-(p-Aminophenyl)ethanol | 5,5 | 40 | Cu₂O | 0,5 | 3,5 | NMP | 350 | 3 | 6,35 / 68 | rotorange, mikrokristallin | >300 |
| 15 | p-Hydroxyphenethylamin | 3,45 | 25 | - | - | - | NMP | 250 | 3 | 7,5 / 80 | braunorange, amorph | >300 |
| 16 | p-Aminobenzolsulfonsäure | 17,3 | 100 | Cu₂O | 1,0 | 7,0 | NMP | 350 | 5 | 6,55 / 65 | rotbraun, kristallin | >300 |

### Analytische Daten zu Beispiel 6:

Elementaranalyse (Gew.-% ber./gef.):
C: 80,0/79,9; H: 3,35/3,3; N: 7,75/7,8; 0: 8,9/9,0;
Masse (FD, 8kV): m/z = 360,2 [M⁺, 100%];
IR (KBr): ν = 2198 (m, C≡N), 1679 (s, C=O), 1648 (s, C=O) cm⁻¹;
UV/VIS (CHCl₃): λₘₐₓ (ε) = 471 (40110), 500 (52300) nm.

### Analytische Daten zu Beispiel 7:

Elementaranalyse (Gew.-% ber./gef.):
C: 81,7/81,5; H: 6,65/6,7; N: 5,45/5,5; 0: 6,2/6,3;
Masse (FD, 8kV): m/z = 514,4 [M⁺, 100%];
IR (KBr): ν = 2200 (m, C=N), 1680 (s, C=O), 1649 (s, C=O) cm⁻¹;
UV/VIS (CHCl₃) : λₘₐₓ (ε) = 472 (37340), 501 (48060) nm.

### Analytische Daten zu Beispiel 8:

Elementaranalyse (Gew.-% ber./gef.):
C: 81,35/81,3; H: 5,95/5,9; N: 5,95/6,0; O: 6,75/6,8;
Masse (FD, 8kV): m/z = 472,5 [M⁺, 100%];
IR (KBr): ν = 2199 (m, C≡N), 1681 (s, C=O), 1648 (s, C=O) cm⁻¹;
UV/VIS (CHCl₃): λₘₐₓ (ε) = 473 (37860), 502 (50120) nm.

### Analytische Daten zu Beispiel 9:

Elementaranalyse (Gew.-% ber./gef.):
C: 83,0/83,2; H: 5,15/5,1; N: 5,55/5,5; 0: 6,3/6,2;
Masse (FD, 8kV): m/z = 506,4 [M⁺, 100%];
IR (KBr): ν = 2202 (m, C≡N), 1682 (s, C=O), 1651 (s, C=O) cm⁻¹;
UV/VIS (CHCl₃): λₘₐₓ (ε) = 474 (36420), 504 (45550) nm.

### Analytische Daten zu Beispiel 10:

Elementaranalyse (Gew.-% ber./gef.):
C: 83,1/83,2; H: 5,65/5,6; N: 5,25/5,2; 0: 6,0/6,0;
Masse (FD, 8kV): m/z = 534,4 [M⁺, 100%];
IR (KBr): ν = 2200 (m, C≡N), 1683 (s, C=O), 1652 (s, C=O) cm⁻¹;
UV/VIS (CHCl₃): λₘₐₓ (ε) = 473 (35040), 505 (42990) nm.

### Analytische Daten zu Beispiel 11:

Elementaranalyse (Gew.-% ber./gef.):
C: 79,8/79,8; H: 3,9/3,9; N: 6,0/6,0; 0: 10,3/10,3;
Masse (FD, 8kV): m/z = 466,3 [M⁺, 100%];
IR (KBr): ν = 2200 (m, C≡N), 1680 (s, C=O), 1650 (s, C=O) cm⁻¹;
UV/VIS (CHCl₃) : λₘₐₓ (ε) = 472 (37080), 502 (47660) nm.

### Analytische Daten zu Beispiel 12:

Elementaranalyse (Gew.-% ber./gef.):
C: 77,75/77,6; H: 4,65/4,7; N: 6,5/6,5; 0: 11,1/11,2;
Masse (FD, 8kV): m/z = 432,4 [M⁺, 100%];
IR (KBr): ν = 2200 (m, C≡N), 1680 (s, C=O), 1650 (s, C=O) cm⁻¹;
UV/VIS (CHCl₃) : λₘₐₓ (ε) = 472 (38220), 501 (50890) nm.

### Analytische Daten zu Beispiel 13:

Elementaranalyse (Gew.-% ber./gef.):
C: 78,2/78,0; H: 5,2/5,2; N: 9,4/9,4; O: 7,2/7,4;
Masse (FD, 8kV): m/z = 445,3 [M⁺, 100%];
IR (KBr): ν = 2199 (m, C≡N), 1678 (s, C=O), 1650 (s, C=O) cm⁻¹;
UV/VIS (CHCl₃): λₘₐₓ (ε) = 471 (37600), 496 (50330) nm.

### Analytische Daten zu Beispiel 14:

Elementaranalyse (Gew.-% ber./gef.):
C: 79,8/79,7; H: 3,9/3,9; N: 6,0/6,0; O: 10,3/10,4;
Masse (FD, 8kV): m/z = 466,4 [M⁺, 100%];
IR (KBr): ν = 2200 (m, C≡N), 1682 (s, C=O), 1652 (s, C=O) cm⁻¹;
UV/VIS (CHCl₃): λₘₐₓ (ε) = 473 (37130), 504 (46220) nm.

### Analytische Daten zu Beispiel 15:

Elementaranalyse (Gew.-% ber./gef.):
C: 79,8/79,6; H: 3,9/3,9; N: 6,0/6,0; O: 10,3/10,5;
Masse (FD, 8kV): m/z = 466,3 [M⁺, 100%];
IR (KBr): ν = 2200 (m, C≡N), 1683 (s, C=O), 1651 (s, C=O) cm⁻¹;
UV/VIS (CHCl₃): λₘₐₓ (ε) = 473 (37200), 503 (46340) nm.

### Analytische Daten zu Beispiel 16:

Elementaranalyse (Gew.-% ber./gef.):
C: 69,3/69,2; H: 2,8/2,8; N: 5,55/5,6; O: 15,95/6,0; S: 6,4/6,4;
Masse (FD, 8kV): m/z = 502,3 [M⁺, 100%];
IR (KBr): ν = 2202 (m, C=N), 1678 (s, C=O), 1651 (s, C=O) cm⁻¹;
UV/VIS (C₂H₂Cl₄): λₘₐₓ (ε) = 473 (36870), 504 (45630) nm.

### d) Herstellung der 9-Cyanoperylen-3,4-dicarbonsäureimide Ib

### Beispiele 17 bis 23

x g (10 mmol) der monofunktionellen 9-Cyanoperylen-3,4-dicarbonsäureimide Ib aus den Beispielen 12 bis 15 wurden unter Rühren in v₁ ml des Verdünnungsmittels VM und v₂ ml der Base B gelöst und auf die gewünschte Reaktionstemperatur T°C erwärmt. Dann wurden langsam eine Lösung von y g (Y mmol) des Carbonsäurechlorids V in v₃ ml des Verdünnungsmittels VM zugetropft und die Reaktionsmischung t h bei der Reaktionstemperatur nachgerührt. Nach Beendigung der Reaktion wurde das Lösungsmittel unter Vakuum bei 60°C (in Beispiel 17 bei 50°C) destillativ entfernt, der Rückstand wurde in möglichst wenig Chloroform gelöst und einer Säulenfiltration über Kieselgel als stationärer und Chloroform als mobiler Phase unterworfen. Abschließend wurde das Lösungsmittel im Vakuum abgezogen.

Weitere Einzelheiten zu diesen Versuchen sowie deren Ergebnisse sind in Tabelle 3 zusammengestellt.

Dabei bedeuten:
- DMF:: N,N-Dimethylformamid
- DBU:: Diazabicycloundecen
- ASC:: Acrylsäurechlorid
- MASC:: Methacrylsäurechlorid

**Tabelle 3**

| Bsp. | x [g] | Ia aus Bsp | v₁ [ml] | Verdm. VM | v₂ [ml] | Base B | v₃ [ml] | y [g] | Y [mmol] | Säurechl. V | t [h] | T [°C] | Ausbeute [g /%] | Aussehen |
|---|---|---|---|---|---|---|---|---|---|---|---|---|---|---|
| 17 | 4,3 | 12 | - | Dioxan | 100 | Pyridin | 50 | 1,35 | 15 | ASC | 2 | 55 | 2,8 / 58 | rotbraun, amorph |
| 18 | 4,3 | 12 | - | Dioxan | 100 | Pyridin | 50 | 1,35 | 13 | MASC | 1,5 | 80 | 3,95 / 79 | braunrot, mikrokrist. |
| 19 | 4,3 | 12 | 100 | Dioxan | 5 | DBU | 50 | 1,35 | 13 | MASC | 1,5 | 80 | 3,5 / 70 | braunrot, mikrokrist. |
| 20 | 4,3 | 12 | 100 | DMF | 10 | Pyridin | 30 | 1,6 | 15 | MASC | 1,5 | 80 | 3,65 / 73 | braunrot, amorph |
| 21 | 4,45 | 13 | - | Dioxan | 100 | Pyridin | 50 | 2,1 | 20 | MASC | 1,5 | 80 | 3,8 / 74 | braun, amorph |
| 22 | 4,65 | 14 | - | Dioxan | 150 | Pyridin | 50 | 2,1 | 20 | MASC | 1,5 | 80 | 4,35 / 81 | rotorange, mikrokrist. |
| 23 | 4,65 | 15 | - | Dioxan | 150 | Pyridin | 50 | 2,6 | 25 | MASC | 2 | 80 | 4,45 / 83 | braunorange, mikrokrist. |

### Analytische Daten zu Beispiel 17:

Elementaranalyse (Gew.-% ber./gef.):
C: 76,55/76,5; H: 4,55/4,5; N: 5,75/5,8; 0: 13,15/13,2;
Masse (FD, 8kV): m/z = 486,4 [M⁺, 100%];
IR (KBr): ν = 2200 (m, C≡N), 1680 (s, C=O), 1650 (s, C=O) cm⁻¹;
UV/VIS (CHCl₃): λₘₐₓ (ε) = 472 (37550), 501 (49980) nm.

### Analytische Daten zu Beispiel 18:

Elementaranalyse (Gew.-% ber./gef.):
C: 76,8/76,8; H: 4,8/4,8; N: 5,6/5,6; O: 12,8/12,8;
Masse (FD, 8kV): m/z = 500,4 [M⁺, 100%];
IR (KBr): v= 2200 (m, C=N), 1680 (s, C=O), 1649 (s, C=O) cm⁻¹;
UV/VIS (CHCl₃): λₘₐₓ (ε) = 472 (36970), 501 (49560) nm.

### Analytische Daten zu Beispiel 21:

Elementaranalyse (Gew.-% ber./gef.):
C: 77,15/77,0; H: 5,3/5,3; N: 8,2/8,25; O: 9,35/9,45;
Masse (FD, 8kV): m/z = 513,5 [M⁺, 100%];
IR (KBr): ν = 2198 (m, C=N), 1682 (s, C=O), 1651 (s, C=O) cm⁻¹;
UV/VIS (CHCl₃): λₘₐₓ (ε) = 472 (36440), 502 (48980) nm.

### Analytische Daten zu Beispiel 22:

Elementaranalyse (Gew.-% ber./gef.):
C: 78,65/78,5; H: 4,15/4,2; N: 5,25/5,3; O: 11,95/12,0;
Masse (FD, 8kV): m/z = 534,5 [M⁺, 100%];
IR (KBr): ν = 2200 (m, C≡N), 1682 (s, C=O), 1651 (s, C=O) cm⁻¹;
UV/VIS (CHCl₃): λₘₐₓ (ε) = 473 (35090), 504 (46720) nm.

### Analytische Daten zu Beispiel 23:

Elementaranalyse (Gew.-% ber./gef.):
C: 78,65/78,4; H: 4,15/4,2; N: 5,25/5,3; O: 11,95/12,1;
Masse (FD, 8kV): m/z = 534,4 [M⁺, 100%];
IR (KBr): ν = 2200 (m, C≡N), 1683 (s, C=O), 1650 (s, C=O) cm⁻¹;
UV/VIS (CHCl₃): λₘₐₓ (ε) = 473 (34990), 503 (46530) nm.

### Herstellung der 9-Cyanoperylen-3,4-dicarbonsäureimide Ic

### Beispiele 24 bis 30

Eine Mischung aus x g (0,1 mol) 9-Bromperylen-3,4-dicarbonsäureimid VI, y g (Y mol) Kupfer(I)cyanid und v ml Verdünnungsmittel V sowie 50 g Pyridin als Katalysator in Beispiel 30 wurde unter Rühren in einer Stickstoffatmosphäre t h auf die gewünschte Reaktionstemperatur T°C erhitzt. Nach dem Abkühlen der Reaktionsmischung wurde entweder mit dem gleichen Volumen Methanol (Beispiele 24, 25, 27, 29) oder aber mit 40 Vol.-% einer Ethanol/Wasser-Mischung im Volumenverhältnis 1:1 (Beispiele 26, 28, 30) verdünnt, der ausgefallene Niederschlag wurde abfiltriert, zur Zersetzung überschüssigen Kupfer(I)cyanids 1 h bei 60°C in einer Mischung aus 1000 ml Wasser, 150 ml konz. Salzsäure und 100 g Eisen(III)- chlorid gerührt, erneut filtriert, der Rückstand mit heißem Wasser bis zum neutralen, fast farblosen Ablauf gewaschen und bei 120°C im Vakuum getrocknet.

Das so hergestellte Rohprodukt wurde zur Reinigung in v' ml 80°C warmem N-Methylpyrrolidon (NMP) gelöst, mit 10 g Aktivkohle versetzt, 30 min bei dieser Temperatur nachgerührt und heiß filtriert. Nach dem Abkühlen auf 60°C wurde das Produkt durch Zusatz von 500 ml 65 vol.-%igem Ethanol ausgefällt, 3 h bei 60°C nachgerührt, langsam auf Raumtemperatur abgekühlt, abfiltriert, mit Ethanol NMP-frei gewaschen und getrocknet.

Weitere Einzelheiten zu diesen Versuchen sowie deren Ergebnisse sind in Tabelle 4 zusammengestellt.

Dabei bedeuten:
- NMP:: N-Methylpyrrolidon
- DMAc:: N,N-Dimethylacetamid

**Tabelle 4**

| Bsp | x [g] | VI | y [g] | Y [mol] | Verdm. V | t [h] | T [°C] | V' [ml] | Ausbeute [g / %] | Aussehen | Smp. [°C] |
|---|---|---|---|---|---|---|---|---|---|---|---|
| 24 | 56,8 | 9-Brom-N-dodecyl-perylendicarboximid | 22,4 | 0,25 | Sulfolan | 1 | 275 | 500 | 42,7 / 83 | rotbraun, mikrokrist. | 220 - 221 |
| 25 | 52,6 | 9-Brom-N-(5-nonyl)-perylendicarboximid | 22,4 | 0,25 | Sulfolan | 1 | 275 | 500 | 35,4 / 75 | rotorange, amorph | 203 - 204 |
| 26 | 48,2 | 9-Brom-N-cyclohexyl-perylendicarboximid | 11,6 | 0,13 | NMP | 3 | 200 | 450 | 33,4 / 78 | rotorange, amorph | >300 |
| 27 | 56,0 | 9-Brom-N-(2,6-diiso-propylphenyl)perylen-dicarboximid | 22,4 | 0,25 | Sulfolan | 1 | 275 | 700 | 40,5 / 80 | orangerot, mikrokrist. | >300 |
| 28 | 56,0 | 9-Brom-N-(2,6-diiso-propylphenyl)perylen-dicarboximid | 11,6 | 0,13 | NMP | 3 | 200 | 675 | 37,9 / 75 | orangerot, mikrokrist. | >300 |
| 29 | 58,8 | 9-Brom-N-(2,5-di-tert.-butylphenyl)-perylendicarboximid | 22,4 | 0,25 | Sulfolan | 1 | 275 | 600 | 43,3 / 81 | orange, mikrokrist. | >300 |
| 30 | 46,6 | 9-Brom-N-(p-meth-oxyben-zyl)perylen-dicarboximid | 17,9 | 0,20 | DMAC | 4 | 165 | 650 | 35,9 / 77 | orangerot, mikrokrist. | >300 |

### Analytische Daten zu Beispiel 26:

Elementaranalyse (Gew.-% ber./gef.):
C: 81,3/81,1; H: 4,7/4,7; N: 6,55/6,6; O: 7,45/7,6;
Masse (FD, 8kV): m/z = 428,2 [M⁺, 100%];
IR (KBr): ν = 2200 (m, C≡N), 1684 (s, C=O), 1649 (s, C=O) cm⁻¹;
UV/VIS (CHCl₃) : λₘₐₓ (ε) = 475 (37100), 502 (48530) nm.

## Patentansprüche

1. 9-cyanosubstituierte Perylen-3,4-dicarbonsäuremonoimide der allgemeinen Formel I 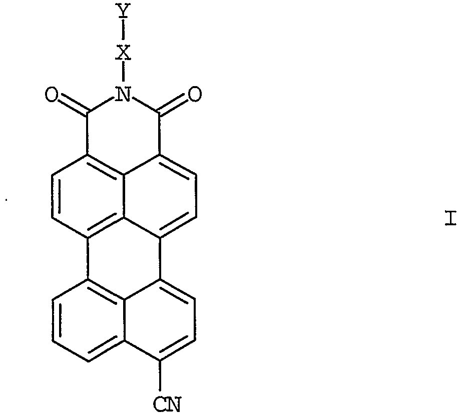 in der die Variablen folgende Bedeutung haben:
X eine chemische Bindung;
C₁-C₃₀-Alkylen, dessen Kohlenstoffkette durch eine oder mehrere Gruppierungen -O-, -S-, -NR¹-, -CO-und/oder -SO₂- unterbrochen sein kann und das durch -COOR¹, -SO₃R¹, Cyano, C₁-C₆-Alkoxy, Aryl, das durch C₁-C₁₈-Alkyl und/oder C₁-C₆-Alkoxy substituiert sein kann, und/oder einen über ein Stickstoffatom gebundenen, 5- bis 7-gliedrigen heterocyclischen Rest, der weitere Heteroatome enthalten und aromatisch sein kann, ein- oder mehrfach substituiert sein kann;
C₅-C₈-Cycloalkylen, dessen Kohlenstoffgerüst durch eine oder mehrere Gruppierungen -O-, -S-, -NR¹-, -CO- und/oder -SO₂- unterbrochen und/oder das ein- oder mehrfach durch C₁-C₁₂-Alkyl, -COOR¹, -SO₃R¹, Cyano und/oder C₁-C₆-Alkoxy substituiert sein kann;
Arylen oder Hetarylen, das jeweils ein- oder mehrfach durch C₁-C₁₈-Alkyl, C₁-C₆-Alkoxy, Cyano, -COOR¹, -SO₃R¹, -CONH-R¹ und/oder -NH-COR¹ substituiert sein kann;
C₁-C₂₀-Alkylarylen oder -hetarylen, dessen Alkylengruppe jeweils durch eine oder mehrere Gruppierungen -O-, -S-, -NR¹-, -CO- und/oder -SO₂- unterbrochen sein kann und das jeweils durch -COOR¹, -SO₃R¹, -CONHR¹, -NHCOR¹, Cyano, C₁-C₁₈-Alkyl, C₁-C₆-Alkoxy und/oder einen über ein Stickstoffatom gebundenen, 5- bis 7-gliedrigen heterocyclischen Rest, der weitere Heteroatome.enthalten und aromatisch sein kann, ein- oder mehrfach substituiert sein kann;
Aryl- oder Hetaryl-C₁-C₂₀-alkylen, dessen Alkylengruppe jeweils durch eine oder mehrere Gruppierungen -O-, -S-, -NR¹-, -CO- und/oder -SO₂- unterbrochen sein kann und das jeweils durch -COOR¹, -SO₃R¹, -CONHR¹, -NHCOR¹, Cyano, C₁-C₁₈-Alkyl, C₁-C₆-Alkoxy und/oder einen über ein Stickstoffatom gebundenen, 5- bis 7-gliedrigen heterocyclischen Rest, der weitere Heteroatome enthalten und aromatisch sein kann, ein- oder mehrfach substituiert sein kann;
Y eine funktionelle Gruppe Y' oder eine polymerisierbare Gruppe P;
oder
X-Y gemeinsam einen Rest R;
Y' Amino, Hydroxy, -COOH, -SO₃H, Chlor oder Brom;
P einen Rest der allgemeinen Formel II 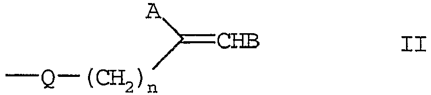
A, B unabhängig voneinander Wasserstoff, C₁-C₆-Alkyl, Phenyl oder gemeinsam einen Cyclopenten- oder Cyclohexenring, der die Doppelbindung, an die A und B gebunden sind, enthält;
Q eine chemische Bindung;
eine Gruppierung -O-, -NR²-, -S-, -OCO-, -OCOO-, -OCONR³-, -NR³CO-, -NR³COO-, -NR³CONR⁴-, -CO-, -COO-, -CONR³-, -SO₂-O-, -SO₂NR³-, -O-SO₂- oder -NR³SO₂-;
n 0, 1, 2 oder 3;
R Wasserstoff;
C₁-C₃₀-Alkyl, dessen Kohlenstoffkette durch eine oder mehrere Gruppierunen -O-, -S-, -NR¹-, -CO- und/oder -SO₂- unterbrochen sein kann und das durch Cyano, C₁-C₆-Alkoxy, Aryl, das durch C₁-C₁₈-Alkyl und/oder C₁-C₆-Alkoxy substituiert sein kann, und/ oder einen über ein Stickstoffatom gebundenen 5-bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₆-Alkyl ein- oder mehrfach substituiert sein kann;
Aryl oder Hetaryl, das jeweils durch C₁-C₁₈-Alkyl, C₁-C₆-Alkoxy, Cyano, -CONHR⁵, -NHCOR⁵ und/oder Aryl- oder Hetarylazo, das jeweils durch C₁-C₁₀-Alkyl, C₁-C₆-Alkoxy und/oder Cyano substituiert sein kann, ein- oder mehrfach substituiert sein kann;
R¹ Wasserstoff oder C₁-C₆-Alkyl;
R² Wasserstoff, C₁-C₆-Alkyl, Aryl, Aryl-C₁-C₆-alkyl, C₁-C₆-Alkylcarbonyl, Arylcarbonyl oder Formyl;
R³, R⁴ unabhängig voneinander Wasserstoff; C₁-C₆-Alkyl; Aryl oder Aryl-C₁-C₆-alkyl, das jeweils durch Hydroxy, Halogen, C₁-C₆-Alkyl und/oder C₁-C₆-Alkoxy substituiert sein kann;
R⁵ Wasserstoff; C₁-C₁₈-Alkyl; Aryl oder Hetaryl, das jeweils durch C₁-C₆-Alkyl, C₁-C₆-Alkoxy, Halogen, Hydroxy, Carboxy und/oder Cyano substituiert sein kann.

2. Perylen-3,4-dicarbonsäuremonoimide der allgemeinen Formel I nach Anspruch 1, in der die Variablen folgende Bedeutung haben:
X C₁-C₃₀-Alkylen, dessen Kohlenstoffkette durch eine oder mehrere Gruppierungen -O- und/oder -CO- unterbrochen sein kann und das durch -COOR¹, Cyano, C₁-C₆-Alkoxy und/oder Aryl, das durch C₁-C₁₈-Alkyl und/oder C₁-C₆-Alkoxy substituiert sein kann, ein- oder mehrfach substituiert sein kann;
C₅-C₈-Cycloalkylen, das ein- oder mehrfach durch C₁-C₁₂-Alkyl, -COOR¹, Cyano und/oder C₁-C₆-Alkoxy substituiert sein kann;
Arylen oder Hetarylen, das jeweils ein- oder mehrfach durch C₁-C₁₈-Alkyl, C₁-C₆-Alkoxy, Cyano, -COOR¹, -CONHR¹ und/oder -NHCOR¹ substituiert sein kann;
C₁-C₂₀-Alkylarylen oder -hetarylen, dessen Alkylengruppe jeweils durch eine oder mehrere Gruppierungen -O- und/oder -CO- unterbrochen sein kann und das jeweils durch -COOR¹, Cyano, C₁-C₁₈-Alkyl und/oder C₁-C₆-Alkoxy ein- oder mehrfach substituiert sein kann;
Aryl- oder Hetaryl-C₁-C₂₀-alkylen, dessen Alkylengruppe jeweils durch eine oder mehrere Gruppierungen -0- und/ oder -CO- unterbrochen sein kann und das jeweils durch -COOR¹, Cyano, C₁-C₁₈-Alkyl und/oder C₁-C₆-Alkoxy ein- oder mehrfach substituiert sein kann;
Y eine funktionelle Gruppe Y' oder eine polymerisierbare Gruppe P;
oder
X-Y gemeinsam einen Rest R;
Y' Amino, Hydroxy, -COOH oder Brom;
P einen Rest der allgemeinen Formel II 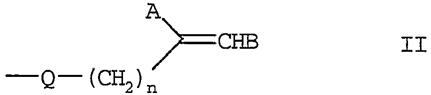
A, B unabhängig voneinander Wasserstoff, C₁-C₆-Alkyl, Phenyl oder gemeinsam einen Cyclopenten- oder Cyclohexenring, der die Doppelbindung, an die A und B gebunden sind, enthält;
Q eine chemische Bindung;
eine Gruppierung -O-, -NR²-, -OCO-, -NR³CO-, -COO- oder -CONR³-;
n 0, 1, 2 oder 3;
R Wasserstoff;
C₁-C₃₀-Alkyl, dessen Kohlenstoffkette durch eine oder mehrere Gruppierungen -O-, -NR¹- und/oder -CO- unterbrochen sein kann und das durch Cyano, C₁-C₆-Alkoxy, Aryl, das durch C₁-C₁₈-Alkyl und/oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O- und/oder -NR¹- unterbrochen und/oder das durch C₁-C₆-Alkyl ein- oder mehrfach substituiert sein kann;
Aryl oder Hetaryl, das jeweils durch C₁-C₁₈-Alkyl, C₁-C₆-Alkoxy, Cyano und/oder Aryl- oder Hetarylazo, das jeweils durch C₁-C₁₀-Alkyl, C₁-C₆-Alkoxy und/oder Cyano substituiert sein kann, ein- oder mehrfach substituiert sein kann;
R¹ Wasserstoff oder C₁-C₆-Alkyl;
R² Wasserstoff, C₁-C₆-Alkyl, Aryl, Aryl-C₁-C₆-alkyl;
R³ Wasserstoff; C₁-C₆-Alkyl; Aryl oder Aryl-C₁-C₆-alkyl, das jeweils durch Hydroxy, C₁-C₆-Alkyl und/oder C₁-C₆-Alkoxy substituiert sein kann.

3. Verfahren zur Herstellung von Perylen-3,4-dicarbonsäuremonoimiden der allgemeinen Formel Ia 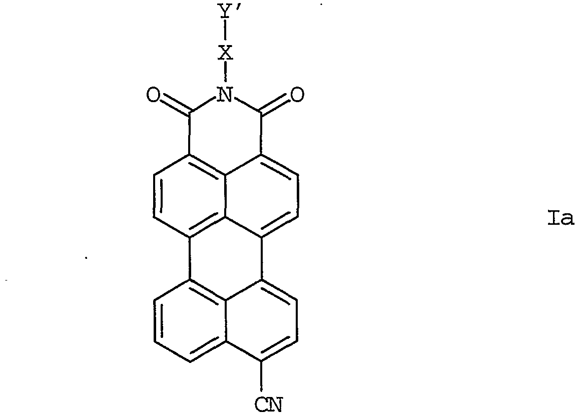 in der X und Y' die in Anspruch 1 angegebene Bedeutung haben oder X-Y' gemeinsam einen der in Anspruch 1 definierten Reste R bedeuten, **dadurch gekennzeichnet, daß** man
a) Perylen-3,4-dicarbonsäureanhydrid in konzentrierter Schwefelsäure oder einer aliphatischen Monocarbonsäure mit elementarem Brom in der 9-Position bromiert,
b) das in Schritt a) erhaltene 9-Bromperylen-3,4-dicarbonsäureanhydrid in einem hochsiedenden inerten Verdünnungsmittel, gegebenenfalls unter Zusatz einer stickstoffbasischen Verbindung oder eines Stickstoffheterocyclus als Katalysator, mit Kupfer(I)cyanid im Überschuß umsetzt und
c) das in Schritt b) erhaltene 9-Cyanoperylen-3,4-dicarbonsäureanhydrid in Wasser oder einem inerten organischen Lösungsmittel, gegebenenfalls unter Zusatz eines Imidierungskatalysators, mit einem primären Amin der allgemeinen Formel IV
Y'-X-NH₂ IV
zu dem gewünschten 9-Cyanoperylen-3,4-dicarbonsäuremonoimid der Formel Ia umsetzt.

4. Verfahren zur Herstellung von Perylen-3,4-dicarbonsäuremonoimiden der allgemeinen Formel Ib 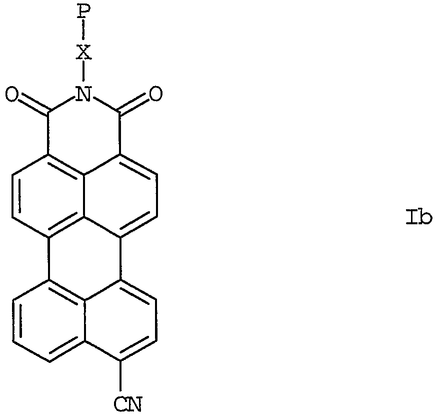 in der X die in Anspruch 1 angegebene Bedeutung hat und P einen der in Anspruch 1 definierten Reste der Formel II, in der Q für -OCO- oder -NHCO- steht, bedeutet, **dadurch gekennzeichnet, daß** man ein Perylen-3,4-dicarbonsäuremonoimid der Formel Ia gemäß Anspruch 3, in der Y' Amino oder Hydroxy bedeutet, in einem inerten aprotischen Verdünnungsmittel unter Zusatz einer Stickstoffbase mit einem Carbonsäurechlorid der allgemeinen Formel V 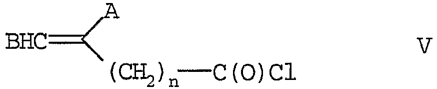 in der die Variablen die in Anspruch 1 angegebene Bedeutung haben, umsetzt.

5. Verfahren zur Herstellung von Perylen-3,4-dicarbonsäuremonoimiden der allgemeinen Formel Ic 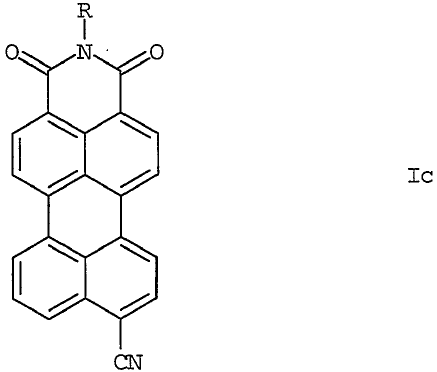 in der R die in Anspruch 1 angegebene Bedeutung hat, **dadurch gekennzeichnet, daß** man ein 9-Bromperylen-3,4-dicarbonsäuremonoimid der allgemeinen Formel VI 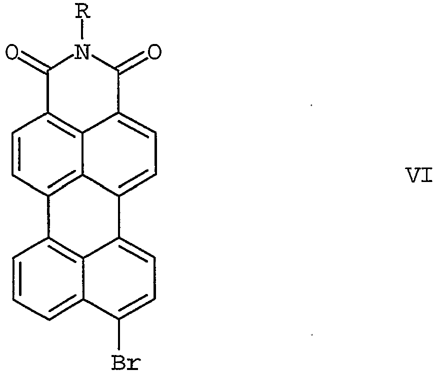 durch Umsetzung mit Kupfer(I)cyanid in Substanz oder in einem hochsiedenden inerten Verdünnungsmittel, gegebenenfalls unter Zusatz einer stickstoffbasischen Verbindung oder eines Stickstoffheterocyclus als Katalysator, in das gewünschte 9-Cyanoperylen-3,4-dicarbonsäuremonoimid der Formel Ic überführt.

6. In 9-Position substituierte Perylen-3,4-dicarbonsäureanhydride der allgemeinen Formel III 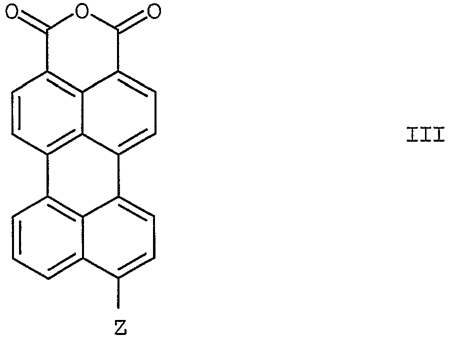 in der Z Brom oder Cyano bedeutet.

7. Verfahren zur Herstellung von 9-Bromperylen-3,4-dicarbonsäureanhydrid der Formel IIIa 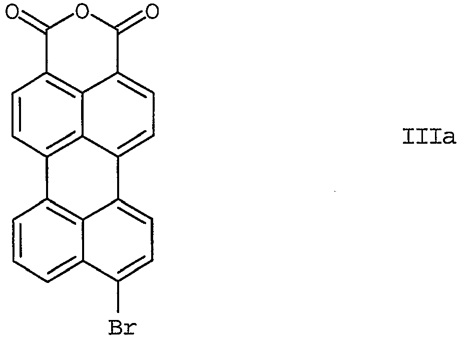 **dadurch gekennzeichnet, daß** man Perylen-3,4-dicarbonsäureanhydrid in konzentrierter Schwefelsäure oder einer aliphatischen Monocarbonsäure mit elementarem Brom selektiv in der 9-Position bromiert.

8. Verfahren zur Herstellung von 9-Cyanoperylen-3,4-dicarbonsäureanhydrid der Formel IIIb 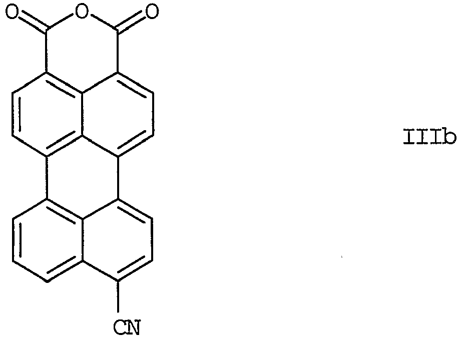 **dadurch gekennzeichnet, daß** man 9-Bromperylen-3,4-dicarbonsäureanhydrid in einem hochsiedenden inerten Verdünnungsmittel, gegebenenfalls unter Zusatz einer stickstoffbasischen Verbindung oder eines Stickstoffheterocyclus als Katalysator, mit Kupfer(I)cyanid im Überschuß umsetzt.

9. Verwendung von 9-cyanosubstituierten Perylen-3,4-dicarbonsäuremonoimiden der allgemeinen Formel I gemäß Anspruch 1 oder 2 zur Einfärbung von hochmolekularen organischen und anorganischen Materialien.

10. Verwendung nach Anspruch 9, **dadurch gekennzeichnet, daß** Kunststoffe, Lacke, Druckfarben, anorganisch-organischen Composite und oxidischen Schichtsysteme eingefärbt werden.

11. Verwendung von 9-cyanosubstituierten Perylen-3,4-dicarbonsäuremonoimiden der allgemeinen Formel I gemäß Anspruch 1 oder 2 als Dispergierhilfsmittel, Pigmentadditive für organische Pigmente und Zwischenprodukte für die Herstellung von Fluoreszenzfarbmitteln und Pigmentadditiven.

12. Verwendung von 9-cyanosubstituierten Perylen-3,4-dicarbonsäuremonoimiden der allgemeinen Formel I gemäß Anspruch 1 oder 2 zur Herstellung im gelben Bereich des elektromagnetischen Spektrums absorbierender und/oder emittierende wäßriger Polymerisatdispersionen und Ink-Jet-Tinten.

13. Verwendung von 9-cyanosubstituierten Perylen-3,4-dicarbonsäuremonoimiden der allgemeinen Formel I gemäß Anspruch 1 oder 2 als farbgebende oder farbkorrigierende Komponente in emissiven und transflektiven Farbfiltern und in retroreflektiven Vorrichtungen.

14. Verwendung von 9-cyanosubstituierten Perylen-3,4-dicarbonsäuremonoimiden der allgemeinen Formel I gemäß Anspruch 1 oder 2 als Photoleiter in der Elektrophotographie, als Emitter in Elektro- und Chemilumineszenzanwendungen, als Aktivkomponenten in der Fluoreszenzkonversion, in Biolumineszenzarrays und in der Photovoltaik und als Laserfarbstoffe.

## Claims

1. A 9-cyano-substituted perylene-3,4-dicarboxylic monoimide of the general formula I 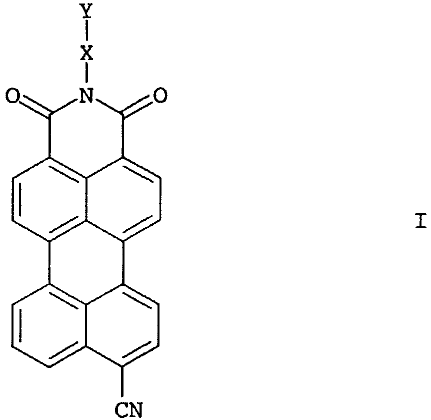 where the variables are defined as follows:
X is a chemical bond;
C₁-C₃₀-alkylene whose carbon chain may be interupted by one or more -O-, -S-, -NR¹-, -CO- and/or -SO₂- moieties, and which may be substituted by -COOR¹, -SO₃R¹, cyano, C₁-C₆-alkoxy, aryl which may be substituted by C₁-C₁₈-alkyl and/or C₁-C₆-alkoxy, and/or be mono- or polysubstituted by a 5- to 7-membered heterocyclic radical which is bonded via a nitrogen atom and may contain further heteroatoms and be aromatic;
C₅-C₈-cycloalkylene whose carbon framework may be interrupted by one or more -O-, -S-, -NR¹-, -CO- and/or -SO₂- moieties and/or may be mono- or polysubstituted by C₁-C₁₂-alkyl, -COOR¹, -SO₃R¹, cyano and/or C₁-C₆-alkoxy;
arylene or hetarylene, each of which may be mono- or polysubstituted by C₁-C₁₈-alkyl, C₁-C₆-alkoxy, cyano, -COOR¹, -SO₃R¹, -CONH-R¹ and/or -NH-COR¹;
C₁-C₂₀-alkylarylene or -hetarylene whose alkylene group may in each case be interrupted by one or more -O-, -S-, -NR¹-, -CO- and/or -SO₂- moieties and which may in each case be mono- or polysubstituted by -COOR¹, -SO₃R¹, -CONHR¹, -NHCOR¹, cyano, C₁-C₁₈-alkyl, C₁-C₆-alkoxy and/or a 5- to 7-membered heterocyclic radical which is bonded via a nitrogen atom and may contain further heteroatoms and be aromatic;
aryl- or hetaryl-C₁-C₂₀-alkylene, whose alkylene group may in each case be interrupted by one or more -O-, -S-, -NR¹-, -CO- and/or -SO₂- moieties and which may each be mono- or polysubstituted by -COOR¹, -SO₃R¹, -CONHR¹, -NHCOR¹, cyano, C₁-C₁₈-alkyl, C₁-C₆-alkoxy and/or a 5- to 7-membered heterocyclic radical which is bonded via a nitrogen atom and may contain further heteroatoms and be aromatic;
Y is a functional group Y' or a polymerizable group P;
or
X-Y together is an R radical;
Y' is amino, hydroxyl, -COOH, -SO₃H, chlorine or bromine;
P is a radical of the general formula II 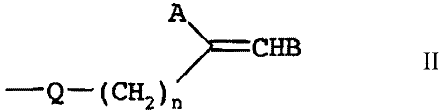
A, B are each independently hydrogen, C₁-C₆-alkyl or phenyl, or are together a cyclopentene or cyclohexene ring which contains the double bond to which A and B are bonded;
Q is a chemical bond;
an -O-, -NR¹-, -S-, -OCO-, -OCOO-, -OCONR³-, -NR³CO-, -NR³COO-, -NR³CONR⁴-, -CO-, -COO-, -CONR³-, -SO₂-O-, -SO₂NR³-, -NR³SO₂- -O-SO₂- or moiety;
n is 0, 1, 2 or 3;
R is hydrogen;
C₁-C₃₀-alkyl whose carbon chain may be interupted by one or more -O-, -S-, -NR¹-, -CO- and/or -SO₂- moieties, and which may be substituted by cyano, C₁-C₆-alkoxy, aryl which may be substituted by C₁-C₁₈-alkyl and/or C₁-C₆-alkoxy, and/or be mono-
or polysubstituted by a 5- to 7-membered heterocyclic radical which is bonded via a nitrogen atom and may contain further heteroatoms and be aromatic;
C₅-C₈-cycloalkyl whose carbon framework may be interrupted by one or more -O-, -S- and/or -NR¹- moieties and/or may be mono- or polysubstituted by C₁-C₆-alkyl;
aryl or hetaryl, each of which may be mono- or polysubstituted by C₁-C₁₈-alkyl, C₁-C₆-alkoxy, cyano, -CONHR⁵, -NHCOR⁵ and/or aryl- or hetarylazo, each of which may be substituted by C₁-C₁₀-alkyl, C₁-C₆-alkoxy and/or cyano;
R¹ is hydrogen or C₁-C₆-alkyl;
R² is hydrogen, C₁-C₆-alkyl, aryl, aryl-C₁-C₆-alkyl, C₁-C₆-alkylcarbonyl, arylcarbonyl or formyl;
R³, R⁴ are each independently hydrogen; C₁-C₆-alkyl; aryl or aryl-C₁-C₆-alkyl, each of which may be substituted by hydroxyl, halogen, C₁-C₆-alkyl and/or C₁-C₆-alkoxy;
R⁵ is hydrogen; C₁-C₁₈-alkyl; aryl or hetaryl, each of which may be substituted by C₁-C₆-alkyl, C₁-C₆-alkoxy, halogen, hydroxyl, carboxyl and/or cyano.

2. A perylene-3,4-dicarboxylic monoimide of the general formula I according to claim 1, in which the variables are defined as follows:
X is C₁-C₃₀-alkylene, whose carbon chain may be interupted by one or more -O- and/or -CO- moieties, and which may be substituted by -COOR¹, cyano, C₁-C₆-alkoxy and/or aryl which may be substituted by C₁-C₁₈-alkyl and/or C₁-C₆-alkoxy;
C₅-C₈-cycloalkylene which may be mono- or polysubstituted by C₁-C₁₂-alkyl, -COOR¹, cyano and/or C₁-C₆-alkoxy;
arylene or hetarylene, each of which may be mono- or polysubstituted by C₁-C₁₈-alkyl, C₁-C₆-alkoxy, cyano, -COOR¹, -CONH-R¹ and/or -NHCOR¹;
C₁-C₂₀-alkylarylene or -hetarylene whose alkylene group may in each case be interrupted by one or more -O- and/or -CO- moieties and which may in each case be mono- or polysubstituted by -COOR¹, cyano, C₁-C₁₈-alkyl and/or C₁-C₆-alkoxy;
aryl- or hetaryl-C₁-C₂₀-alkylene, whose alkylene group may in each case be interrupted by one or more -O- and/or -CO- moieties and which may in each case be mono- or polysubstituted by -COOR¹, cyano, C₁-C₁₈-alkyl and/or C₁-C₆-alkoxy;
Y is a functional group Y' or a polymerizable group P;
or
X-Y together is an R radical;
Y' is amino, hydroxyl, -COOH or bromine;
P is a radical of the general formula II 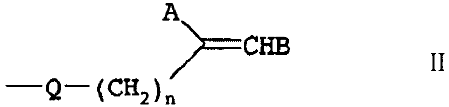
A, B are each independently hydrogen, C₁-C₆-alkyl or phenyl, or are together a cyclopentene or cyclohexene ring which contains the double bond to which A and B are bonded;
Q is a chemical bond; a -O-, -NR²-, -OCO-, -NR³CO-, -COO- or -CONR³-moiety;
n is 0, 1, 2 or 3;
R is hydrogen;
C₁-C₃₀-alkyl whose carbon chain may be interupted by one or more -O-, -NR¹- and/or -CO- moieties, and which may be substituted by cyano, C₁-C₆-alkoxy, aryl which may be substituted by C₁-C₁₈-alkyl and/or C₁-C₆-alkoxy, and/or be mono- or polysubstituted by a 5- to 7-membered heterocyclic radical which is bonded via a nitrogen atom and may contain further heteroatoms and be aromatic;
C₅-C₈-cycloalkyl whose carbon framework may be interrupted by one or more -O- and/or -NR¹- moieties and/or may be mono- or polysubstituted by C₁-C₆-alkyl;
aryl or hetaryl, each of which may be mono- or polysubstituted by C₁-C₁₈-alkyl, C₁-C₆-alkoxy, cyano and/or aryl- or hetarylazo, each of which may be substituted by C₁-C₁₀-alkyl, C₁-C₆-alkoxy and/or cyano;
R¹ is hydrogen or C₁-C₆-alkyl;
R² is hydrogen, C₁-C₆-alkyl, aryl, aryl-C₁-C₆-alkyl;
R³ is hydrogen; C₁-C₆-alkyl; aryl or aryl-C₁-C₆-alkyl, each of which may be substituted by hydroxyl, C₁-C₆-alkyl and/or C₁-C₆-alkoxy.

3. A process for preparing perylene-3,4-dicarboxylic monoimides of the general formula Ia 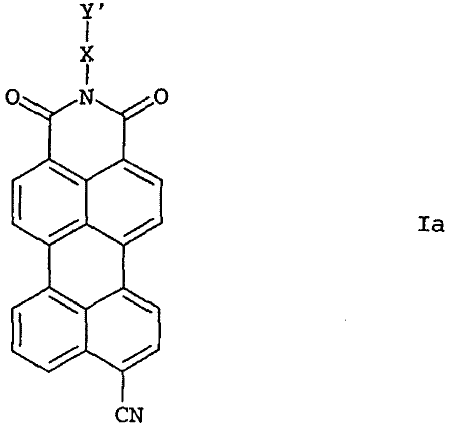 where X and Y' are as defined in claim 1 or X-Y' together are one of the R radicals defined in claim 1, which comprises
a) brominating perylene-3,4-dicarboxylic anhydride in the 9-position using elemental bromine in concentrated sulfuric acid or an aliphatic monocarboxylic acid,
b) reacting the 9-bromoperylene-3,4-dicarboxylic anhydride obtained in step a) with copper(I) cyanide in excess in a high-boiling inert diluent, optionally with the addition of a basic nitrogen compound or of a nitrogen heterocycle as a catalyst, and
reacting the 9-cyanoperylene-3,4-dicarboxylic anhydride obtained in step
b) with a primary amine of the general formula IV
**Y' -X-NH₂** **IV**
in water or an inert organic solvent, optionally with the addition of an imidation catalyst, to give the desired 9-cyanoperylene-3,4-dicarboxylic monoimide of the formula Ia.

4. A process for preparing perylene-3,4-dicarboxylic monoimides of the general formula Ib 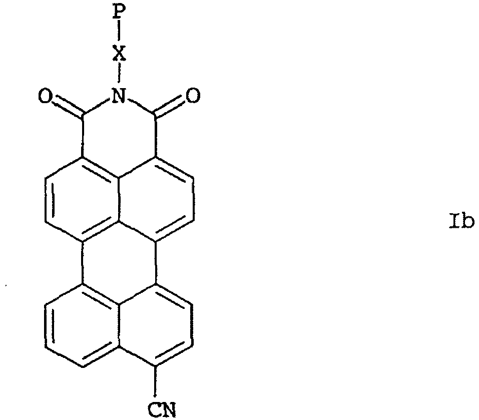 where X is as defined in claim 1 and P is one of the radicals of the formula II defined in claim 1 where Q is -OCO- or -NHCO-, which comprises reacting a perylene-3,4-dicarboxylic monoimide of the formula Ia as defined in claim 3 where Y' is amino or hydroxyl with a carbonyl chloride of the general formula V 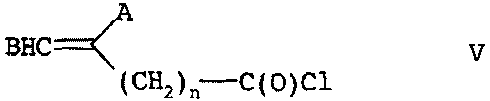 where the variables are as defined in claim 1 in an inert aprotic diluent, with the addition of a nitrogen base.

5. A process for preparing perylene-3,4-dicarboxylic monoimides of the general formula Ic 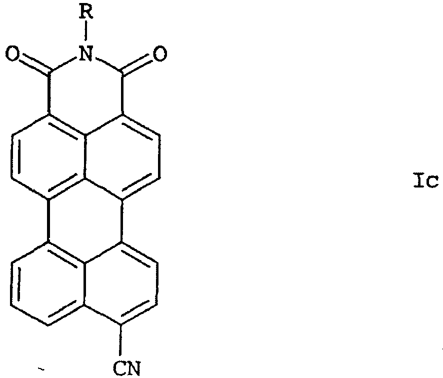 where R is as defined in claim 1, which comprises converting a 9-bromoperylene-3,4-dicarboxylic monoimide of the general formula VI 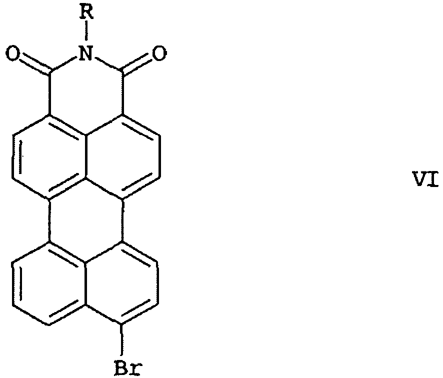 to the desired 9-cyanoperylene-3,4-dicarboxylic monoimide of the formula Ic by reacting with copper(I) cyanide without a diluent or in a high-boiling inert diluent, optionally with the addition of a basic nitrogen compound or of a nitrogen heterocycle as a catalyst.

6. A perylene-3,4-dicarboxylic anhydride, substituted in the 9-position, of the general formula III 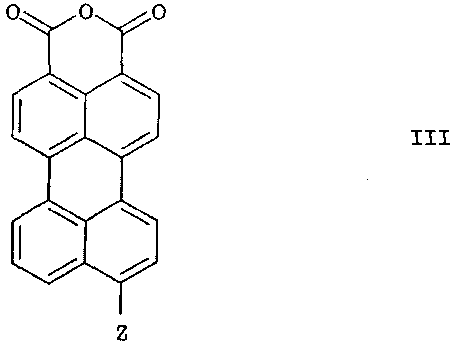 where Z is bromine or cyano.

7. A process for preparing 9-bromoperylene-3,4-dicarboxylic anhydride of the formula IIIa 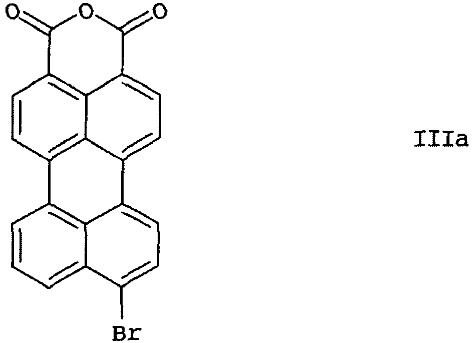 which comprises selectively brominating perylene-3,4- dicarboxylic anhydride in the 9-position with elemental bromine in concentrated sulfuric acid or an aliphatic monocarboxylic acid.

8. A process for preparing 9-cyanoperylene-3,4-dicarboxylic anhydride of the formula IIIb 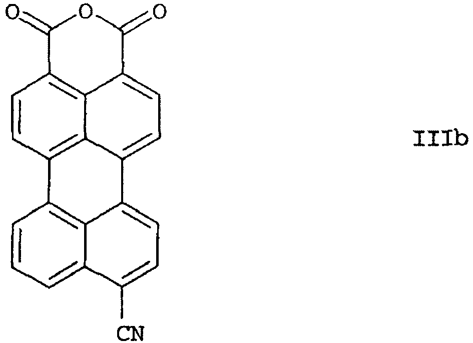 which comprises reacting 9-bromoperylene-3,4-dicarboxylic anhydride with copper(I) cyanide in excess in a high-boiling inert diluent, optionally with the addition of a basic nitrogen compound or of a nitrogen heterocycle as a catalyst.

9. The use of 9-cyano-substituted perylene-3,4-dicarboxylic monoimides of the general formula I according to claim 1 or 2 for coloring high molecular weight organic and inorganic materials.

10. The use according to claim 9, wherein plastics, paints, printing inks, inorganic-organic composites and oxidic layer systems are colored.

11. The use of 9-cyano-substituted perylene-3,4-dicarboxylic monoimides of the general formula I according to claim 1 or 2 as dispersants, pigment additives for organic pigments and intermediates for the preparation of fluorescent dyes and pigment additives.

12. The use of 9-cyano-substituted perylene-3,4-dicarboxylic monoimides of the general formula I according to claim 1 or 2 for producing aqueous polymer dispersions and inkjet inks absorbing and/or emitting in the yellow region of the electromagnetic spectrum.

13. The use of 9-cyano-substituted perylene-3,4-dicarboxylic monoimides of the general formula I according to claim 1 or 2 as a coloring or color-correcting component in emissive and transflective color filters and in retroreflective components.

14. The use of 9-cyano-substituted perylene-3,4-dicarboxylic monoimides of the general formula I according to claim 1 or 2 as photoconductors in electrophotography, as emitters in electroluminescence and chemiluminescence applications, as active components in fluorescence conversion, in bioluminescence arrays and in photovoltaics and as a laser dye.

## Revendications

1. Pérylène-3,4-dicarboximides 9-cyano-substitués de formule générale I 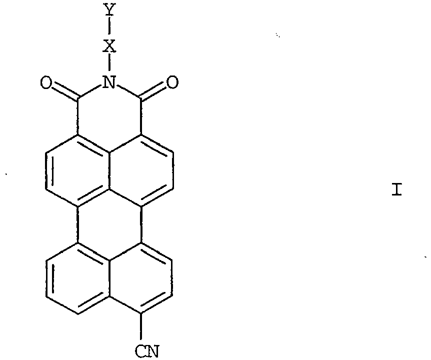 dans laquelle les variables ont les significations suivantes :
X est une liaison chimique ;
un groupe alkylène en C₁-C₃₀, dont la chaîne carbonée peut être interrompue par un ou plusieurs groupements -O-, -S-, -NR¹-, -CO- et/ou -SO₂-, et qui peut être substituée par un ou plusieurs substituants -COOR¹, -SO₃R¹, cyano, alcoxy en C₁-C₆, aryle qui peut être substitué par un ou plusieurs substituants alkyle en C₁-C₁₈ et/ou alcoxy en C₁-C₆, et/ou un radical hétérocyclique à 5 à 7 chaînons, lié par l'intermédiaire d'un atome d'azote, et qui peut contenir d'autres hétéroatomes et être aromatique ;
un groupe cycloalkylène en C₅-C₈, dont le squelette carboné peut être interrompu par un ou plusieurs groupements -O-, -S-, -NR¹-, -CO- et/ou -SO₂-, et/ou qui peut être substitué par un ou plusieurs substituants alkyle en C₁-C₁₂, -COOR¹, -SO₃R¹, cyano et/ou alcoxy en C₁-C₆ ;
un groupe arylène ou hétéroarylène, dont chacun peut être substitué par un ou plusieurs substituants alkyle en C₁-C₁₈, alcoxy en C₁-C₆, cyano, -COOR¹, -SO₃R¹, -CONH-R¹ et/ou -NH-COR¹ ;
un groupe (alkyle en C₁-C₂₀) -arylène ou -hétéroarylène, le groupe alkylène de chacun d'eux pouvant être interrompu par un ou plusieurs groupements -O-, -S-, -NR¹-, -CO- et/ou -SO₂-, et dont chacun peut être substitué par un ou plusieurs substituants -COOR¹, -SO₃R¹, -CONHR¹, -NHCOR¹, cyano, alkyle en C₁-C₁₈, alcoxy en C₁-C₆ et/ou un radical hétérocyclique à 5 à 7 chaînons, lié par l'intermédiaire d'un atome d'azote, qui peut contenir d'autres hétéroatomes et être aromatique ;
un groupe aryl- ou hétéroaryl-(alkylène en C₁-C₂₀). le groupe alkylène de chacun d'eux pouvant être interrompu par un ou plusieurs groupements -0-, -S-, -NR¹-, -CO- et/ou -SO₂-, et chacun pouvant être substitué par un ou plusieurs substituants -COOR¹-, -SO₃R¹, -CONHR¹, -NHCOR¹, cyano, alkyle en C₁-C₁₈, alcoxy en C₁-C₆ et/ou un radical hétérocyclique à 5 à 7 chaînons, lié par l'intermédiaire d'un atome d'azote, qui peut contenir d'autres hétéroatomes et être aromatique ;
Y est un groupe fonctionnel Y' ou un groupe polymérisable P ;
ou
X-Y forment ensemble un radical R ;
Y' est un groupe amino, hydroxy, -COOH, -SO₃H, chloro ou bromo ;
P est un radical de formule générale II 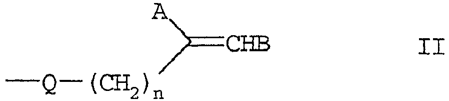
A,B représentent chacun indépendamment de l'autre un atome d'hydrogène, un groupe alkyle en C₁-C₆, phényle, ou forment ensemble un noyau cyclopentène ou cyclohexène, qui contient la double liaison à laquelle A et B sont liés ;
Q est une liaison chimique ;
un groupement -O-, -NR²-, -S-, -OCO-, -OCOO-, -OCONR³-, -NR³CO-, -NR³COO-, -NR³CONR⁴-, -CO-, -COO-, -CONR³-, -SO₂-O-, -SO₂NR³-, -O-SO₂- ou -NR³SO₂- ;
n vaut 0, 1, 2 ou 3 ;
R est un atome d'hydrogène ;
un groupe alkyle en C₁-C₃₀, dont la chaîne carbonée peut être interrompue par un ou plusieurs groupements-O-, -S-, -NR¹-, -CO- et/ou -SO₂-, et qui peut être substitué par un ou plusieurs substituants cyano, alcoxy en C₁-C₆, aryle qui peut être substitué par un ou plusieurs substituants alkyle en C₁-C₁₈ et/ou alcoxy en C₁-C₆, et/ou un radical hétérocyclique à 5 à 7 chaînons, lié par l'intermédiaire d'un atome d'azote, qui peut contenir d'autres hétéroatomes et être aromatique ;
un groupe cycloalkyle en C₅-C₈, dont le squelette carboné peut être interrompu par un ou plusieurs groupements -O-, -S- et/ou -NR¹-, et/ou qui peut être substitué par un ou plusieurs substituants alkyle en C₁-C₆ ;
aryle ou hétéroaryle, dont chacun peut être substitué par un ou plusieurs substituants alkyle en C₁-C₁₈, alcoxy en C₁-C₆, cyano, -CONHR⁵, -NHCOR⁵ et/ou aryl- ou hétéroarylazo, dont chacun peut être substitué par un ou plusieurs substituants alkyle en C₁-C₁₀, alcoxy en C₁-C₆ et/ou cyano ;
R¹ est un atome d'hydrogène ou un groupe alkyle en C₁-C₆ ;
R² est un atome d'hydrogène, un groupe alkyle en C₁-C₆, aryle, aryl-(alkyle en C₁-C₆), (alkyle en C₁-C₆)carbonyle, arylcarbonyle ou formyle ;
R³, R⁴ représentent chacun indépendamment de l'autre un atome d'hydrogène ; un groupe alkyle en C₁-C₆ ; un groupe aryle ou aryl-(alkyle en C₁-C₆), dont chacun peut être substitué par un ou plusieurs substituants hydroxy, halogéno, alkyle en C₁-C₆ et/ou alcoxy en C₁-C₆ ;
R⁵ est un atome d'hydrogène ; un groupe alkyle en C₁-C₁₈ ; aryle ou hétéroaryle, dont chacun peut être substitué par un ou plusieurs substituants alkyle en C₁-C₆, alcoxy en C₁-C₆, halogéno, hydroxy, carboxy et/ou cyano.

2. Pérylène-3,4-dicarboximides de formule générale I selon la revendication 1, dans laquelle les variables ont les significations suivantes :
X est un groupe alkylène en C₁-C₃₀, dont la chaîne carbonée peut être interrompue par un ou plusieurs groupements -O- et/ou -CO- et qui peut être substitué par un ou plusieurs substituants -COOR¹, cyano, alcoxy en C₁-C₆ et/ou aryle qui peut être substitué par un ou plusieurs substituants alkyle en C₁-C₁₈ et/ou alcoxy en C₁-C₆ ;
un groupe cycloalkylène en C₅-C₈, qui peut être substitué par un ou plusieurs substituants alkyle en C₁-C₁₂, -COOR¹, cyano et/ou alcoxy en C₁-C₆ ;
un groupe arylène ou hétéroarylène, dont chacun peut être substitué par un ou plusieurs substituants alkyle en C₁-C₁₈, alcoxy en C₁-C₆, cyano, -COOR¹, -CONHR¹ et/ou -NHCOR¹ ;
un groupe (alkyle en C₁-C₂₀) arylène ou -hétéroarylène, le groupe arylène de chacun d'eux pouvant être interrompu par un ou plusieurs groupements -O- et/ou -CO-, et dont chacun peut être substitué par un ou plusieurs substituants -COOR¹, cyano, alkyle en C₁-C₁₈ et/ou alcoxy en C₁-C₆ ;
un groupe aryl- ou hétéroaryl-(alkylène en C₁-C₂₀), le groupe alkylène de chacun d'eux pouvant être interrompu par un ou plusieurs groupements -O- et/ou -CO-, et dont chacun peut être substitué par un ou plusieurs substituants -COOR¹, cyano, alkyle en C₁-C₁₈ et/ou alcoxy en C₁-C₆ ;
Y est un groupe fonctionnel Y' ou un groupe polymérisable P ;
ou
X-Y forment ensemble un radical R ;
Y' est un groupe amino, hydroxy, -COOH ou bromo ;
P est un radical de formule générale II 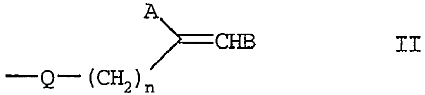 A,B représentent chacun indépendamment de l'autre un atome d'hydrogène, un groupe alkyle en C₁-C₆, phényle, ou forment ensemble un noyau cyclopentène ou cyclohexène, qui contient la double liaison à laquelle A et B sont liés ;
Q est une liaison chimique ;
un groupement -O-, -NR²-, -OCO-, -NR³CO-, -COO- ou -CONR³- ;
n vaut 0, 1, 2 ou 3 ;
R est un atome d'hydrogène ;
un groupe alkyle en C₁-C₃₀, dont la chaîne carbonée peut être interrompue par un ou plusieurs groupements -O-, -NR¹- et/ou -CO-, et qui être substitué par un ou plusieurs substituants cyano, alcoxy en C₁-C₆, aryle qui peut être substitué par un ou plusieurs substituants alkyle en C₁-C₁₈ et/ou alcoxy en C₁-C₆, et/ou un radical hétérocyclique à 5 à 7 chaînons, liés par l'intermédiaire d'un atome d'azote, qui peut contenir d'autres hétéroatomes et qui peut être aromatique ;
un groupe cycloalkyle en C₅-C₈, dont le squelette carboné peut être interrompu par un ou plusieurs groupements -O- et/ou -NR¹-, et/ou qui peut être substitué par un ou plusieurs substituants alkyle en C₁-C₆ ;
aryle ou hétéroaryle, dont chacun peut être substitué par un ou plusieurs substituants alkyle en C₁-C₁₈, alcoxy en C₁-C₆, cyano et/ou aryl- ou hétéroarylazo, dont chacun peut être substitué par un ou plusieurs substituants alkyle en C₁-C₁₀, alcoxy en C₁-C₆ et/ou cyano ;
R¹ est un atome d'hydrogène ou un groupe alkyle en C₁-C₆ ;
R² est un atome d'hydrogène, un groupe alkyle en C₁-C₆, aryle, aryl (alkyle en C₁-C₆) ;
R³ est un atome d'hydrogène ; un groupe alkyle en C₁-C₆ ; aryle ou aryl-(alkyle en C₁-C₆), dont chacun peut être substitué par un ou plusieurs substituants hydroxy, alkyle en C₁-C₆ et/ou alcoxy en C₁-C₆.

3. Procédé de préparation de pérylène-3,4-dicarboximides de formule générale Ia 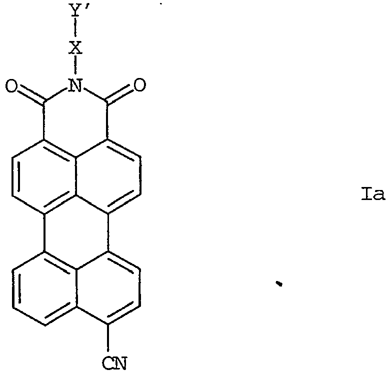 dans laquelle X et Y' ont les significations données dans la revendication 1, ou X-Y' forment ensemble l'un des radicaux R définis dans la revendication 1, **caractérisé en ce que**
a) on brome avec du brome élémentaire en position 9 de l'anhydride pérylène-3,4-dicarboxylique dans de l'acide sulfurique concentré ou un autre acide monocarboxylique aliphatique,
b) on fait réagir avec du cyanure de cuivre(I) en excès l'anhydride 9-bromopérylène-3,4-dicarboxylique obtenu dans l'étape a) dans un diluant inerte à haut point d'ébullition, éventuellement avec addition d'un composé à base azotée ou un composé hétérocyclique azoté servant de catalyseur, et
c) on fait réagir l'anhydride 9-cyanopérylène-3,4-dicarboxylique obtenu dans l'étape b) dans de l'eau ou dans un solvant organique inerte, éventuellement avec addition d'un catalyseur d'imidation, avec une amine primaire de formule générale IV
Y'-X-NH₂ IV
pour obtenir le 9-cyanopérylène-3,4-dicarboximide souhaité de formule Ia.

4. Procédé de préparation de pérylène-3,4-dicarboximides de formule générale Ib 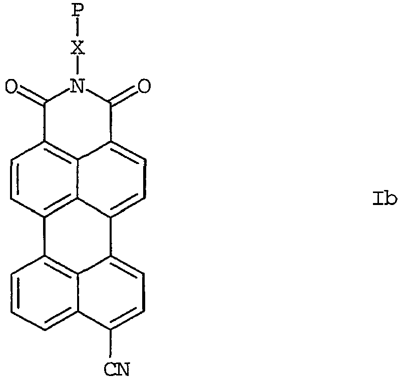 dans laquelle X a les significations données dans la revendication 1 et P représente l'un des radicaux de formule II définis dans la revendication 1, dans laquelle Q représente -OCO- ou -NHCO-, **caractérisé en ce qu'**on fait réagir un pérylène-3,4-dicarboximide de formule Ia selon la revendication 3, dans laquelle Y' est un groupe amino ou hydroxy, dans un diluant aprotique inerte, avec addition d'une base azotée, avec un chlorure de carbonyle de formule générale V 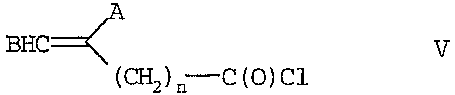 dans laquelle les variables ont les significations données dans la revendication 1.

5. Procédé de préparation de pérylène-3,4-dicarboximides de formule générale Ic 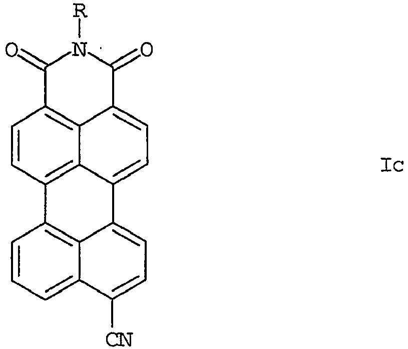 dans laquelle R a les significations données dans la revendication 1, **caractérisé en ce qu'**on convertit en le 9-cyanopérylène-3,4-dicarboximide de formule générale Ic un 9-bromopérylène-3,4-dicarboximide de formule générale VI 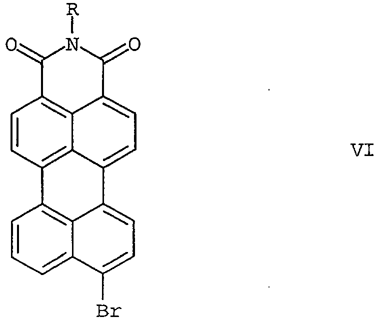 par réaction avec du cyanure de cuivre(I), sans solvant ou dans un diluant inerte à haut point d'ébullition, éventuellement avec addition d'un composé à base azotée ou d'un composé hétérocyclique azoté servant de catalyseur.

6. Anhydrides pérylène-3,4-dicarboxyliques substitués en position 9 de formule générale III 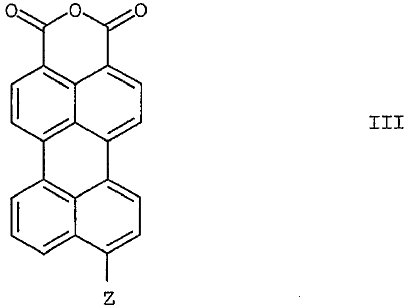 dans laquelle Z représente le groupe bromo ou cyano.

7. Procédé de préparation d'anhydride 9-bromopérylène-3,4-dicarboxylique de formule générale IIIa 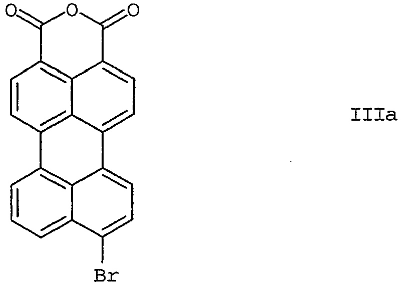 **caractérisé en ce qu'**on brome sélectivement en position 9 avec du brome élémentaire de l'anhydride pérylène-3,4-dicarboxylique dans de l'acide sulfurique concentré ou dans un acide monocarboxylique aliphatique.

8. Procédé de préparation d'anhydride 9-cyanopérylène-3,4-dicarboxylique de formule IIIb 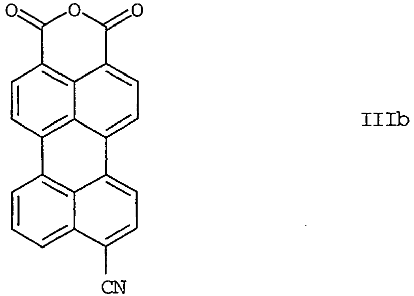 **caractérisé en ce qu'**on fait réagir avec du cyanure de cuivre(I) en excès un anhydride 9-bromopérylène-3,4-dicarboxylique dans un diluant inerte à haut point d'ébullition, éventuellement avec addition d'un composé à base azotée ou d'un composé hétérocyclique azoté servant de catalyseur.

9. Utilisation de pérylène-3,4-dicarboxymides 9-cyano substitués de formule générale I selon la revendication 1 ou 2 pour colorer dans la masse des matériaux organiques ou inorganiques à grande masse moléculaire.

10. Utilisation selon la revendication 9, **caractérisée en ce qu'**on colore dans la masse des matériaux plastiques, des peintures, des encres d'imprimerie, des composites inorganiques-organiques et des systèmes de couches à base d'oxydes.

11. Utilisation de pérylène-3,4-dicarboxymides 9-cyano substitués de formule générale I selon la revendication 1 ou 2, en tant qu'auxiliaires de dispersion, additifs pour pigments organiques et produits intermédiaires pour la préparation de colorants fluorescents et d'additifs pour pigments.

12. Utilisation de pérylène-3,4-dicarboxymides 9-cyano substitués de formule générale I selon la revendication 1 ou 2, pour préparer des dispersions aqueuses de polymères et des encres pour jet d'encre, absorbant et/ou émettant dans le domaine jaune du spectre électromagnétique.

13. Utilisation de pérylène-3,4-dicarboxymides 9-cyano substitués de formule générale I selon la revendication 1 ou 2, en tant que composants chromogènes ou correcteurs de couleur dans des filtres couleurs émissifs ou transflectifs et dans des dispositifs rétro-réfléchissants.

14. Utilisation de pérylène-3,4-dicarboxymides 9-cyano substitués de formule générale I selon la revendication 1 ou 2, en tant que photoconducteurs en électrophotographie, en tant qu'émetteurs dans les applications d'électroluminescence et de chimioluminescence, en tant que composants actifs en conversion de fluorescence, dans les puces à bioluminescence, et en photovoltaïque et en tant que colorants laser.
